# EUROPEAN PATENT APPLICATION

(11) **EP 3 757 103 A1**
(43) Date of publication of application: **30.12.2020**
(21) Application number: 19182797.1
(22) Date of filing: 27.06.2019
(51) Int. Cl.: C07D 417/12, C07D 417/14, A61P 13/00, A61P 25/00, A61P 29/00, A61P 31/00, A61K 31/427

(54) **ANALOGUES OF 3-(5-METHYL-1,3-THIAZOL-2-YL)-N-{(1R)-1-[2-(TRIFLUORO-METHYL)PYRIMIDIN-5-YL]ETHYL}BENZAMIDE FOR THE TREATMENT OF NEUROGENIC DISEASES**

(71) Applicant: Bayer AG, 51373 Leverkusen (DE); Bayer Pharma Aktiengesellschaft, 13353 Berlin (DE)
(72) Inventor: Rottmann, Antje, 13089 Berlin (DE); Fischer, Oliver Martin, 13127 Berlin (DE); Thede, Kai, 10437 Berlin (DE); Herbert, Simon, Anthony, 10249 Berlin (DE); Ganzer, Ursula, 13505 Berlin (DE); Rotgeri, Andrea, 13503 Berlin (DE); Pook, Elisabeth, 42109 Wuppertal (DE)
(74) Representative: BIP Patents

(57) **Abstract**

The present invention covers P2X3 inhibitor compounds of general formula (I) : 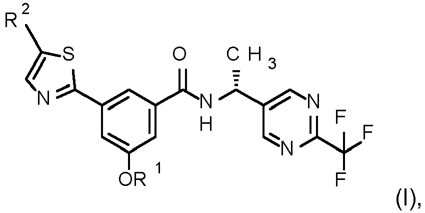 in which R¹ and R² are as defined herein, methods of preparing said compounds, intermediate compounds useful for preparing said compounds, pharmaceutical compositions and combinations comprising said compounds and the use of said compounds for manufacturing pharmaceutical compositions for the treatment or prophylaxis of diseases, in particular of neurogenic disorders, as a sole agent or in combination with other active ingredients.

## Description

The present invention covers compounds of general formula (I) as described and defined herein, methods of preparing said compounds, intermediate compounds useful for preparing said compounds, pharmaceutical compositions and combinations comprising said compounds, and the use of said compounds for manufacturing pharmaceutical compositions for the treatment or prophylaxis of diseases, in particular of neurogenic disorders, as a sole agent or in combination with other active ingredients.

### Background

The present invention relates to 1,3-thiazol-2-yl substituted benzamide compounds of general formula (I) which inhibit P2X3 receptor.
P2X purinoceptor 3 is a protein that in humans is encoded by the P2RX3 gene (Garcia-Guzman M, Stuhmer W, Soto F (Sep 1997). "Molecular characterization and pharmacological properties of the human P2X3 purinoceptor". Brain Res Mol Brain Res 47 (1-2): 59-66). The product of this gene belongs to the family of purinoceptors for ATP. This receptor functions as a ligand-gated ion channel and transduces ATP-evoked nociceptor activation.

P2X purinoreceptors are a family of ligand-gated ion channels that are activated by ATP. To date, seven members of this family have been cloned, comprising P2X1-7 [Burnstock 2013, front Cell Neurosci 7:227]. These channels can exist as homomers and heteromers [Saul 2013, front Cell Neurosci 7:250]. Purines, such as ATP, have been recognized as important neurotransmitters and by acting via their respective receptors they have been implicated in various physiological and pathophysiological roles [Burnstock 1993, Drug Dev Res 28:196-206; Burnstock 2011, Prog Neurobiol 95:229-274; Jiang 2012, Cell Health Cytoskeleton 4:83-101].
Among the P2X family members, in particular the P2X3 receptor has been recognized as an important mediator of nociception [Burnstock 2013, Eur J Pharmacol 716:24-40; North 2003, J Phyiol 554:301-308; Chizh 2000, Pharmacol Rev 53:553-568]. It is mainly expressed in dorsal root ganglia in a subset of nociceptive sensory neurons. During inflammation the expression of the P2X3 receptor is increased, and activation of P2X3 receptor has been described to sensitize peripheral nerves [Fabretti 2013, front Cell Neurosci 7:236].
The prominent role of the P2X3 receptor in nociception has been described in various animal models, including mouse and rat models for acute, chronic and inflammatory pain. P2X3 receptor knock-out mice show a reduced pain response [Cockayne 2000, Nature 407:1011-1015; Souslova 2000, Nature 407:1015-1017]. P2X3 receptor antagonists have been shown to act anti-nociceptive in different models of pain and inflammatory pain [Ford 2012, Purin Signal 8 (Suppl 1):S3-S26]. The P2X3 receptor has also been shown to integrate different nociceptive stimuli. Hyperalgesia induced by PGE2, ET-1 and dopamine have all been shown to be mediated via release of ATP and activation of the P2X3 receptor [Prado 2013, Neuropharm 67:252-258; Joseph 2013, Neurosci 232C: 83-89].
Besides its prominent role in nociception and in pain-related diseases involving both chronic and acute pain, the P2X3 receptor has been shown to be involved in genitourinary, gastrointestinal and respiratory conditions and disorders, including overactive bladder and chronic cough [Ford 2013, front Cell Neurosci 7:267; Burnstock 2014, Purin Signal 10(1):3-50]. ATP-release occurs in these 2 examples from epithelial cells, which in turn activates the P2X3 receptor and induces contraction of bladder and lung muscles respectively leading to premature voiding or cough.
P2X3 subunits do not only form homotrimers but also heterotrimers with P2X2 subunits. P2X3 subunits and P2X2 subunits are also expressed on nerve fibres innervating the tongue, therein taste buds [Kinnamon 2013, front Cell Neurosci 7:264]. In a phyiosological setting, receptors containing P2X3 and/ or P2X2 subunits are involved in the transmission of taste from the tongue (bitter, sweet, salty, umami and sour). Recent data show that while blocking the P2X3 homomeric receptor alone is important to achieve anti-nociceptive efficacy, non-selective blockade of both the P2X3 homomeric receptor and the P2X2/3 heteromeric receptor leads to changes in taste perception which might limit the therapeutic use of non-selective P2X3 and P2X2/3 receptor antagonists [Ford 2014, purines 2014, abstract book p15]. Therefore, compounds that differentiate between P2X3 and P2X2/3 receptors are highly desirable.
Compounds blocking both the P2X3 subunit containing ion channel (P2X3 homomer) as well as the ion channel composed of P2X2 and P2X3 subunit (P2X2/3 heterotrimer) are called P2X3 and P2X2/3 nonselective receptor antagonists [Ford, Pain Manag 2012]. Clinical Phil trials demonstrated that AF-219, a P2X3 antagonist, leads to taste disturbances in treated subjects by affecting taste sensation via the tongue [e.g. Abdulqawi et al, Lancet 2015; Strand et al, 2015 ACR/ARMP Annual Meeting, Abstract 2240]. This side effect has been attributed to the blockade of P2X2/3 channels, i.e. the heterotrimer [A. Ford, London 2015 Pain Therapeutics Conference, congress report]. Knock-out animals deficient for P2X2 and P2X3 subunits show reduced taste sensation and even taste loss [Finger et al, Science 2005], whereas P2X3 subunit single knockouts exhibit a mild or no change in phenotype with respect to taste. Moreover, 2 distinct populations of neurons have been described in the geniculate ganglion expressing either P2X2 and P2X3 subunits or P2X3 subunit alone. In an in vivo setting assessing taste preference towards an artificial sweetener via a lickometer, only at very high free plasma levels (> 100 µM) effects on taste were observed, indicating that rather the P2X2 and P2X3 subunits expressing population plays a major role in taste sensation than the P2X3 subunit expressing population [Vandenbeuch et al, J Physiol. 2015]. Hence, as a modified taste perception has profound effects on the quality of life of patients, P2X3-homomeric receptor-selective antagonists are deemed to be superior towards non-selective receptor antagonists and are considered to represent a solution towards the problem of insufficient patient compliance during chronic treatment as indicated by increased drop-out rates during Phil trials [Strand et al, 2015 ACR/ARMP Annual Meeting, Abstract 2240 and A. Ford, London 2015 Pain Therapeutics Conference, congress report].

Increased sympathetic nervous system (SNS) activity and sympathetic neural factors such as norepinephrine (NE, also known as noradrenaline) are involved in the genesis of cardiovascular disease (CVD) in general (Grassi et al, Circ Res, 2015, 116(6):976-990). Common comorbidities with heart failure (HF) and CVD are also associated with increased sympathetic tone and decreased parasympathetic tone, termed autonomic imbalance. Taken together, clinical studies indicate that patients suffering from autonomic imbalance have decreased exercise tolerance, higher incidence of central sleep apneas, higher incidence of arrhythmias, and increased mortality (Joyner, J Physiol, 2016, 549(14): 4009-4013). Autonomic imbalance is an independent predictor of mortality in HF and CVD patients regardless of the etiology of the condition and is caused by chronic pathological over-activation of afferent inputs such as peripheral chemoreceptors.
Recent preclinical and clinical studies have demonstrated that the carotid body peripheral chemoreflex should be considered as a target for cardiovascular diseases associated with autonomic imbalance (Del Rio et al, J Am Coll Cardiol, 2013, 62(25):2422-2430; McBryde et al, Nat Commun, 2013, 4:2395; Niewinsky et al, Int J Cardiol, 2013, 168(3):2506-2509; Paton et al, Hypertension, 2013, 61(1):5-13; Marcus et al, J Physiol, 2014, 592(2):391-408; Del Rio et al, Exp Physiol, 2015, 100(2):136-142). Chemoreflex hypersensitivity has been demonstrated in animal models of CVD with different etiology including: genetic modifications, chronic intermittent hypoxia, myocardial infarction, rapid ventricular pacing, genetic cardiomyopathy, and pressure overload.
Increased chemoreflex sensitivity is observed in 40-60 % of optimally treated HF patients (Giannoni et al, J Am Coll Cardiol, 2009, 53(21):1975-1980; Niewinski et al, J Card Fail, 2013, 19(6):408-415). Chemoreflex hypersensitivity is also associated with a higher prevalence of unstable ventilatory control during wakefulness, ventilatory insufficiency during exercise, sleep related breathing disorders, Cheyne-Stokes respiration, persistent atrial fibrillation, and paroxysmal ventricular tachycardia, and impaired baroreflex control of blood pressure (Ponikowski et al, Circulation. 2001. 104(5):544-549; Corra et al, Circulation, 2006, 113(1):44-50; Giannoni et al, Clin Sci (Lond). 2008. 114(7):489-497; Despas et al, J Hypertens, 2012, 30(4):753-760; Dempsey and Smith, Adv Exp Med Biol. 2014. 758:343-349; Andrade et al, Biomed Res Int. 2015. 467597; Floras and Ponikowski, Eur Heart J, 2015, 36(30):1974-1982b; Grassi et al, Circ Res, 2015, 116(6):976-990).

In the case of cardiovascular diseases (CVD), neurotransmitter release, including ATP release from Type I and Type II glumus cells of the carotid body (glomus caroticum) is involved in the the physiological response to hypoxia. Recent studies (Pijacka et al, Nat Med, 2016, 22(10): 1151-1159) demonstrate that overexpression of P2X3 in the carotid body of spontaneously hypertensive rats increases tonic activation of the peripheral chemoreflex leading to increased sympathetic nervous system activity and autonomic imbalance (Pijacka et al, Nat Med, 2016, 22(10): 1151-1159). Therefore blockade of P2X3 could be considered as a treatment option for CVD associated with tonically active or hypersensitive peripheral chemoreflex.

1,3-thiazol-2-yl substituted benzamide compounds have been disclosed in WO2016/091776 A1. The compounds disclosed in WO2016/091776 A1 show high P2X3 receptor inhibition and furthermore selectivity over the P2X2/3 receptor.

Further non-published patent applications, i.e. PCT/EP2019/062329 and PCT/EP2019/062332, disclose the use of compounds of WO2016/091776 A1 in the treatment of cardiovascular diseases and of chronic cough.

However, the state of the art described above does not describe the specific thiazole substituted benzamide compounds of general formula (I) of the present invention as defined herein or isomers, enantiomers, diastereomers, racemates, hydrates, solvates, or salts thereof, or a mixture of same, as described and defined herein, and as hereinafter referred to as "compounds of the present invention", and their pharmacological activity.
It has now been found, and this constitutes the basis of the present invention, that the compounds of the present invention have surprising and advantageous properties.
In particular, the compounds of the present invention have surprisingly been found to effectively inhibit P2X3 receptor for which data are given in biological experimental section and may therefore be used for the treatment or prophylaxis of neurogenic disorders, such as pain-related disorders, for example. In addition to that, the compounds of the present invention are characterized by a favourable pharmacological profile, e.g. having in addition to the inhibitory efficacy at the P2X3 receptor and selectivity over the P2X2/3 receptor closed to those described in the prior art a favourable solubility and/ or suitable metabolic stability.

### Description of the Invention

As dissolution, solubility and intestinal permeability govern the rate and extent of drug absorption from solid oral dosage forms, it is highly desirable to improve said physicochemical and pharmacokinetic properties.
A favourable solubility in the meaning of the present invention stands for a better dissolution of the compound at the required dose compared to compounds known from the prior art. The improvement of the dissolution behaviour of compounds results in better intestinal absorption and oral bioavailability of said compounds. A high oral bioavailability in humans of more than 70% ensures the administration of compounds at high dosages if it is desired in order to achieve a therapeutic effect.
A suitable metabolic stability in liver microsomes in the meaning of the present invention means better pharmacokinetic properties in human *in vivo* in terms of a low hepatic clearance which results in a longer half-life and a higher exposure of a compound with the same dose. Lower clearance means a human blood clearance of less than 30% of liver blood flow (lower of approximately 0.4 L/h/kg) leading to a maximum oral bioavailability of 70%.
The exposure of a compound in terms of its blood concentration has to be in the range of its IC80 value in order to achieve a therapeutic effect on diseases, which are depending on P2X3 receptor modulation (e.g. pain). Said relation is based on the assumption that the higher the metabolic stability the longer is the half-life of a compound to keep efficacious blood concentrations during the dosing interval. Hence, as longer the half-life of the compound the lower the dose and the longer the treatment interval is for the patient. A long half-life in humans means more than 12 hours.

In accordance with a first aspect, the present invention covers compounds of general formula (I) 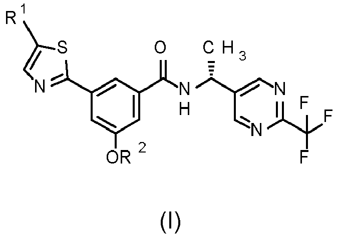 in which
R¹ represents methyl or -COOH,
R² represents C₃-C₄-alkyl optionally substituted with one or two substituents which are the same or different and independently selected from the group consisting of OH and -COOH, or 5-membered heterocycloalkyl having one O atom and optionally substituted at any carbon atom with one or two substituents which are the same or different, and independently selected from the group consisting of oxo and OH,
or stereoisomers, hydrates, solvates, salts thereof, or mixtures of same, as described and defined herein.

### DEFINITIONS

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.
The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen atom. Commonly, it is possible for the number of optional substituents, when present, to be 1, 2, 3, 4 or 5, in particular 1 or 2.
As used herein, the term "one or more", *e.g.* in the definition of the substituents of the compounds of general formula (I) of the present invention, means "1, 2, 3, 4 or 5, particularly 1 or 2".
As used herein, an oxo substituent represents an oxygen atom, which is bound to a carbon atom via a double bond.
The term "comprising" when used in the specification includes "consisting of".
If within the present text any item is referred to as "as mentioned herein", it means that it may be mentioned anywhere in the present text.
The terms as mentioned in the present text have the following meanings:
The term "C₃-C₄-alkyl" means a linear or branched, saturated, monovalent hydrocarbon group having 3 or 4 carbon atoms, e.g. a propyl, isopropyl, butyl, sec-butyl, isobutyl, *tert*-butyl, methylpropyl, or an isomer thereof. Particular, said group is a butyl group. More particular, said group is a methylpropyl group and even more particular a 1-methylpropyl group.
The terms "5-membered heterocycloalkyl" means a monocyclic, saturated heterocycle with 5 ring atoms in total, which contains one or two ring heteroatom O.
Said heterocycloalkyl group, without being limited thereto, can be a tetrahydrofuranyl, 1,3-dioxolanyl, 1,2-oxazolidinyl, or 1,3-oxazolidinyl for example.
It is possible for the compounds of general formula (I) to exist as isotopic variants. The invention therefore includes one or more isotopic variant(s) of the compounds of general formula (I), particularly deuterium-containing compounds of general formula (I).

The term "Isotopic variant" of a compound or a reagent is defined as a compound exhibiting an unnatural proportion of one or more of the isotopes that constitute such a compound.
The term "Isotopic variant of the compound of general formula (I)" is defined as a compound of general formula (I) exhibiting an unnatural proportion of one or more of the isotopes that constitute such a compound.
The expression "unnatural proportion" means a proportion of such isotope which is higher than its natural abundance. The natural abundances of isotopes to be applied in this context are described in "Isotopic Compositions of the Elements 1997", Pure Appl. Chem., 70(1), 217-235, 1998.
Examples of such isotopes include stable and radioactive isotopes of hydrogen, carbon, nitrogen, oxygen, phosphorus, sulfur, fluorine, chlorine, bromine and iodine, such as ²H (deuterium), ³H (tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁵I, ¹²⁹I and ¹³¹I, respectively.
With respect to the treatment and/or prophylaxis of the disorders specified herein the isotopic variant(s) of the compounds of general formula (I) preferably contain deuterium ("deuterium-containing compounds of general formula (I)"). Isotopic variants of the compounds of general formula (I) in which one or more radioactive isotopes, such as ³H or ¹⁴C, are incorporated are useful e.g. in drug and/or substrate tissue distribution studies. These isotopes are particularly preferred for the ease of their incorporation and detectability. Positron emitting isotopes such as ¹⁸F or ¹¹C may be incorporated into a compound of general formula (I). These isotopic variants of the compounds of general formula (I) are useful for in vivo imaging applications. Deuterium-containing and ¹³C-containing compounds of general formula (I) can be used in mass spectrometry analyses in the context of preclinical or clinical studies.
Isotopic variants of the compounds of general formula (I) can generally be prepared by methods known to a person skilled in the art, such as those described in the schemes and/or examples herein, by substituting a reagent for an isotopic variant of said reagent, preferably for a deuterium-containing reagent. Depending on the desired sites of deuteration, in some cases deuterium from D₂O can be incorporated either directly into the compounds or into reagents that are useful for synthesizing such compounds. Deuterium gas is also a useful reagent for incorporating deuterium into molecules. Catalytic deuteration of olefinic bonds and acetylenic bonds is a rapid route for incorporation of deuterium. Metal catalysts (i.e. Pd, Pt, and Rh) in the presence of deuterium gas can be used to directly exchange deuterium for hydrogen in functional groups containing hydrocarbons. A variety of deuterated reagents and synthetic building blocks are commercially available from companies such as for example C/D/N Isotopes, Quebec, Canada; Cambridge Isotope Laboratories Inc., Andover, MA, USA; and CombiPhos Catalysts, Inc., Princeton, NJ, USA.
The term "deuterium-containing compound of general formula (I)" is defined as a compound of general formula (I), in which one or more hydrogen atom(s) is/are replaced by one or more deuterium atom(s) and in which the abundance of deuterium at each deuterated position of the compound of general formula (I) is higher than the natural abundance of deuterium, which is about 0.015%. Particularly, in a deuterium-containing compound of general formula (I) the abundance of deuterium at each deuterated position of the compound of general formula (I) is higher than 10%, 20%, 30%, 40%, 50%, 60%, 70% or 80%, preferably higher than 90%, 95%, 96% or 97%, even more preferably higher than 98% or 99% at said position(s). It is understood that the abundance of deuterium at each deuterated position is independent of the abundance of deuterium at other deuterated position(s).
The selective incorporation of one or more deuterium atom(s) into a compound of general formula (I) may alter the physicochemical properties (such as for example acidity [C. L. Perrin, et al., J. Am. Chem. Soc., 2007, 129, 4490], basicity [C. L. Perrin et al., J. Am. Chem. Soc., 2005, 127, 9641], lipophilicity [B. Testa et al., Int. J. Pharm., 1984, 19(3), 271]) and/or the metabolic profile of the molecule and may result in changes in the ratio of parent compound to metabolites or in the amounts of metabolites formed. Such changes may result in certain therapeutic advantages and hence may be preferred in some circumstances. Reduced rates of metabolism and metabolic switching, where the ratio of metabolites is changed, have been reported (A. E. Mutlib et al., Toxicol. Appl. Pharmacol., 2000, 169, 102). These changes in the exposure to parent drug and metabolites can have important consequences with respect to the pharmacodynamics, tolerability and efficacy of a deuterium-containing compound of general formula (I). In some cases deuterium substitution reduces or eliminates the formation of an undesired or toxic metabolite and enhances the formation of a desired metabolite (e.g. Nevirapine: A. M. Sharma et al., Chem. Res. Toxicol., 2013, 26, 410; Efavirenz: A. E. Mutlib et al., Toxicol. Appl. Pharmacol., 2000, 169, 102). In other cases the major effect of deuteration is to reduce the rate of systemic clearance. As a result, the biological half-life of the compound is increased. The potential clinical benefits would include the ability to maintain similar systemic exposure with decreased peak levels and increased trough levels. This could result in lower side effects and enhanced efficacy, depending on the particular compound's pharmacokinetic/ pharmacodynamic relationship. ML-337 (C. J. Wenthur et al., J. Med. Chem., 2013, 56, 5208) and Odanacatib (K. Kassahun et al., WO2012/112363) are examples for this deuterium effect. Still other cases have been reported in which reduced rates of metabolism result in an increase in exposure of the drug without changing the rate of systemic clearance (e.g. Rofecoxib: F. Schneider et al., Arzneim. Forsch. / Drug. Res., 2006, 56, 295; Telaprevir: F. Maltais et al., J. Med. Chem., 2009, 52, 7993). Deuterated drugs showing this effect may have reduced dosing requirements (e.g. lower number of doses or lower dosage to achieve the desired effect) and/or may produce lower metabolite loads.
A compound of general formula (I) may have multiple potential sites of attack for metabolism. To optimize the above-described effects on physicochemical properties and metabolic profile, deuterium-containing compounds of general formula (I) having a certain pattern of one or more deuterium-hydrogen exchange(s) can be selected. Particularly, the deuterium atom(s) of deuterium-containing compound(s) of general formula (I) is/are attached to a carbon atom and/or is/are located at those positions of the compound of general formula (I), which are sites of attack for metabolizing enzymes such as e.g. cytochrome P₄₅₀.
Where the plural form of the word compounds, salts, polymorphs, hydrates, solvates and the like, is used herein, this is taken to mean also a single compound, salt, polymorph, isomer, hydrate, solvate or the like.
By "stable compound' or "stable structure" is meant a compound that is sufficiently robust to survive isolation to a useful degree of purity from a reaction mixture, and formulation into an efficacious therapeutic agent.
The compounds of the present invention optionally contain one or more asymmetric centres, depending upon the location and nature of the various substituents desired. It is possible that one or more asymmetric carbon atoms are present in the (R) or (S) configuration, which can result in racemic mixtures in the case of a single asymmetric centre, and in diastereomeric mixtures in the case of multiple asymmetric centres. In certain instances, it is possible that asymmetry also be present due to restricted rotation about a given bond, for example, the central bond adjoining two substituted aromatic rings of the specified compounds.
Preferred compounds are those, which produce the more desirable biological activity. Separated, pure or partially purified isomers and stereoisomers or racemic or diastereomeric mixtures of the compounds of the present invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (e.g., HPLC columns using a chiral phase), with or without conventional derivatisation, optimally chosen to maximise the separation of the enantiomers. Suitable HPLC columns using a chiral phase are commercially available, such as those manufactured by Daicel, e.g., Chiracel OD and Chiracel OJ, for example, among many others, which are all routinely selectable. Enzymatic separations, with or without derivatisation, are also useful. The optically active compounds of the present invention can likewise be obtained by chiral syntheses utilizing optically active starting materials.
In order to distinguish different types of isomers from each other reference is made to IUPAC Rules Section E (Pure Appl Chem 45, 11-30, 1976).
The present invention includes all possible stereoisomers of the compounds of the present invention as single stereoisomers, or as any mixture of said stereoisomers, e.g. (R)- or (S)- isomers, in any ratio. Isolation of a single stereoisomer, e.g. a single enantiomer or a single diastereomer, of a compound of the present invention is achieved by any suitable state of the art method, such as chromatography, especially chiral chromatography, for example.
Further, the compounds of the present invention can exist as N-oxides, which are defined in that at least one nitrogen of the compounds of the present invention is oxidised. The present invention includes all such possible N-oxides.
The present invention also covers useful forms of the compounds of the present invention, such as hydrates, solvates, prodrugs, salts, in particular pharmaceutically acceptable salts, and/or co-precipitates.
The compounds of the present invention can exist as a hydrate, or as a solvate, wherein the compounds of the present invention contain polar solvents, in particular water, methanol, or ethanol for example, as structural element of the crystal lattice of the compounds. It is possible for the amount of polar solvents, in particular water, to exist in a stoichiometric or non-stoichiometric ratio. In the case of stoichiometric solvates, *e.g.* a hydrate, hemi-, (semi-), mono-, sesqui-, di-, tri-, tetra-, penta- *etc.* solvates or hydrates, respectively, are possible. The present invention includes all such hydrates or solvates.
Further, it is possible for the compounds of the present invention to exist in free form, e.g. as a free base, or as a free acid, or as a zwitterion, or to exist in the form of a salt. Said salt may be any salt, either an organic or inorganic addition salt, particularly any pharmaceutically acceptable organic or inorganic addition salt, which is customarily used in pharmacy, or which is used, for example, for isolating or purifying the compounds of the present invention.
The term "pharmaceutically acceptable salt" refers to an inorganic or organic acid addition salt of a compound of the present invention. For example, see S. M. Berge, et al. "Pharmaceutical Salts," J. Pharm. Sci. 1977, 66, 1-19.
A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, or "mineral acid", such as hydrochloric, hydrobromic, hydroiodic, sulfuric, sulfamic, bisulfuric, phosphoric, or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, 3-phenylpropionic, pivalic, 2-hydroxyethanesulfonic, itaconic, trifluoromethanesulfonic, dodecylsulfuric, ethanesulfonic, benzenesulfonic, para-toluenesulfonic, methanesulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, or thiocyanic acid, for example.
Further, another suitably pharmaceutically acceptable salt of a compound of the present invention which is sufficiently acidic, is an alkali metal salt, for example a sodium or potassium salt, an alkaline earth metal salt, for example a calcium, magnesium or strontium salt, or an aluminium or a zinc salt, or an ammonium salt derived from ammonia or from an organic primary, secondary or tertiary amine having 1 to 20 carbon atoms, such as ethylamine, diethylamine, triethylamine, ethyldiisopropylamine, monoethanolamine, diethanolamine, triethanolamine, dicyclohexylamine, dimethylaminoethanol, diethylaminoethanol, tris(hydroxymethyl)aminomethane, procaine, dibenzylamine, *N*-methylmorpholine, arginine, lysine, 1,2-ethylenediamine, *N-*methylpiperidine, *N*-methyl-glucamine, *N,N*-dimethyl-glucamine, *N*-ethyl-glucamine, 1,6-hexanediamine, glucosamine, sarcosine, serinol, 2-amino-1,3-propanediol, 3-amino-1,2-propanediol, 4-amino-1,2,3-butanetriol, or a salt with a quarternary ammonium ion having 1 to 20 carbon atoms, such as tetramethylammonium, tetraethylammonium, tetra(n-propyl)ammonium, tetra(*n*-butyl)ammonium, *N*-benzyl-*N,N,N*-trimethylammonium, choline or benzalkonium.
Those skilled in the art will further recognise that it is possible for acid addition salts of the claimed compounds to be prepared by reaction of the compounds with the appropriate inorganic or organic acid via any of a number of known methods. Alternatively, alkali and alkaline earth metal salts of acidic compounds of the present invention are prepared by reacting the compounds of the present invention with the appropriate base via a variety of known methods.
The present invention includes all possible salts of the compounds of the present invention as single salts, or as any mixture of said salts, in any ratio.
In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.
Unless specified otherwise, suffixes to chemical names or structural formulae relating to salts, such as "hydrochloride", "trifluoroacetate", "sodium salt", or "x HCI", "x CF₃COOH", "x Na⁺", for example, mean a salt form, the stoichiometry of which salt form not being specified.
This applies analogously to cases in which synthesis intermediates or example compounds or salts thereof have been obtained, by the preparation and/or purification processes described, as solvates, such as hydrates, with (if defined) unknown stoichiometric composition.
As used herein, the term *"in vivo* hydrolysable ester" means an *in vivo* hydrolysable ester of a compound of the present invention containing a carboxy or hydroxy group, for example, a pharmaceutically acceptable ester which is hydrolysed in the human or animal body to produce the parent acid or alcohol. Suitable pharmaceutically acceptable esters for carboxy include for example alkyl, cycloalkyl and optionally substituted phenylalkyl, in particular benzyl esters, C₁-C₆ alkoxymethyl esters, *e.g.* methoxymethyl, C₁-C₆ alkanoyloxymethyl esters, *e.g.* pivaloyloxymethyl, phthalidyl esters, C₃-C₈ cycloalkoxy-carbonyloxy-C₁-C₆ alkyl esters, *e.g.* 1-cyclohexylcarbonyloxyethyl ; 1,3-dioxolen-2-onylmethyl esters, *e.g.* 5-methyl-1,3-dioxolen-2-onylmethyl ; and C₁-C₆-alkoxycarbonyloxyethyl esters, *e.g.* 1-methoxycarbonyloxyethyl, it being possible for said esters to be formed at any carboxy group in the compounds of the present invention.
An *in vivo* hydrolysable ester of a compound of the present invention containing a hydroxy group includes inorganic esters such as phosphate esters and [alpha]-acyloxyalkyl ethers and related compounds which as a result of the *in vivo* hydrolysis of the ester breakdown to give the parent hydroxy group. Examples of [alpha]-acyloxyalkyl ethers include acetoxymethoxy and 2,2-dimethylpropionyloxymethoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. The present invention covers all such esters.
Furthermore, the present invention includes all possible crystalline forms, or polymorphs, of the compounds of the present invention, either as single polymorph, or as a mixture of more than one polymorph, in any ratio.
Moreover, the present invention also includes prodrugs of the compounds according to the invention. The term "prodrugs" here designates compounds which themselves can be biologically active or inactive, but are converted (for example metabolically or hydrolytically) into compounds according to the invention during their residence time in the body.

In accordance with a second embodiment of the first aspect, the invention covers compounds of formula (I) wherein R¹ represents methyl,
and stereoisomers, hydrates, solvates, and salts thereof, and mixtures of same.
In accordance with a third embodiment of the first aspect, the present invention covers compounds of general formula (I), *supra,* wherein R² represents C₄-alkyl optionally substituted with one or two groups of OH, or 5-membered heterocycloalkyl having one or two O atom and optionally substituted at any carbon atom with one or two substituents which are the same or different, and selected from the group oxo, and OH, and stereoisomers, hydrates, solvates, and salts thereof, and mixtures of same.

In accordance with a further embodiment of the first aspect, the present invention covers compounds of general formula (I), *supra,* wherein R² represents C₃-alkyl optionally substituted identically or differently by one or two groups of OH or -COOH, or 5-membered heterocycloalkyl having one O atom and optionally substituted at any carbon atom with one or two substituents which are the same or different, and selected from the group consisting of oxo, and OH,
and stereoisomers, hydrates, solvates, and salts thereof, and mixtures of same.

In accordance with a further embodiment of the first aspect, the present invention covers compounds of general formula (I), *supra,* wherein R² represents C₃-alkyl optionally substituted with OH and COOH,
and stereoisomers, hydrates, solvates, and salts thereof, and mixtures of same.

In accordance with a further embodiment of the first aspect, the present invention covers compounds of general formula (I), supra, wherein R² represents C₄-alkyl optionally substituted with two OH groups,
and stereoisomers, hydrates, solvates, and salts thereof, and mixtures of same.

In accordance with a further embodiment of the first aspect, the present invention covers compounds of general formula (I), *supra,* wherein R² represents

-CH(CH₂OH)(CH₂)₂OH,

and stereoisomers, hydrates, solvates, and salts thereof, and mixtures of same.

In accordance with a further embodiment of the first aspect, the present invention covers compounds of general formula (I), *supra,* wherein R² represents tetrahydrofuranyl optionally substituted at any carbon atom with one or two substituents which are the same or different, and selected from the group oxo and OH,
and stereoisomers, hydrates, solvates, and salts thereof, and mixtures of same.

In accordance with a further embodiment of the first aspect, the present invention covers compounds of general formula (I), *supra,* wherein R² represents a tetrahydrofuranyl group of formula (II) 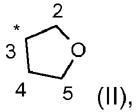
optionally substituted at any carbon atom with one OH, and
* indicates the point of attachment of said group with the rest of the molecule via the oxygen atom, supra,
and stereoisomers, hydrates, solvates, and salts thereof, and mixtures of same.

In accordance with a further embodiment of the first aspect, the present invention covers compounds of general formula (I), supra, wherein R² represents a tetrahydrofuranyl group of formula (II) optionally substituted with OH at carbon atom 5 of said tetrahydrofuranyl group,
and stereoisomers, hydrates, solvates, and salts thereof, and mixtures of same.

In accordance with a further embodiment of the first aspect, the present invention covers compounds of general formula (I), *supra,* in which:
R¹ represents methyl, and
R² represents tetrahydrofuranyl optionally substituted at any carbon atom with one OH,
and stereoisomers, hydrates, solvates, and salts thereof, and mixtures of same.

In accordance with a further embodiment of the first aspect, the present invention covers compounds of general formula (I), supra, in which:
R¹ represents methyl, and
R² represents tetrahydrofuranyl optionally substituted with OH at carbon atom 5,
and stereoisomers, hydrates, solvates, and salts thereof, and mixtures of same.

In accordance with a further embodiment of the first aspect, the present invention covers compounds of general formula (I), supra, in which:
R¹ represents methyl, and
R² represents CH(CH₂OH)(CH₂)₂OH,
and stereoisomers, hydrates, solvates, and salts thereof, and mixtures of same.

In accordance with a further embodiment of the first aspect, the present invention covers compounds of general formula (I), supra, in which:
R¹ represents methyl, and
R² represents CH(CH₂OH)(CH₂COOH),
and stereoisomers, hydrates, solvates, and salts thereof, and mixtures of same.

In accordance with a further embodiment of the first aspect, the present invention covers compounds of general formula (I), supra, in which:
R¹ represents -COOH, and
R² represents tetrahydrofuranyl optionally substituted at any carbon atom with one OH
and stereoisomers, hydrates, solvates, and salts thereof, and mixtures of same.

In accordance with a further embodiment of the first aspect, the present invention covers compounds of general formula (I), supra, in which:
R¹ represents -COOH, and
R² represents unsubstitued tetrahydrofuranyl,
and stereoisomers, hydrates, solvates, and salts thereof, and mixtures of same.

In a particular further embodiment of the first aspect, the present invention covers combinations of two or more of the above-mentioned embodiments under the heading "further embodiments of the first aspect of the present invention."
The present invention covers any sub-combination within any embodiment or aspect of the present invention of compounds of general formula (I), *supra.*
The present invention covers any sub-combination within any embodiment or aspect of the present invention of intermediate compounds of general formula.
The present invention covers the compounds of general formula (I) which are disclosed in the Example Section of this text, *infra.*

The compounds according to the invention of general formula (I) can be prepared starting from compound (III). The synthesis of compound (III), i.e. 3-(5-Methyl-1,3-thiazol-2-yl)-5-[(3R)-tetrahydrofuran-3-yloxy]-N-{(1R)-1-[2-(trifluoro-methyl)pyrimidin-5-yl]ethyl}benzamide, is described in WO2016/091776 A1.
The compounds of formula (I) can be prepared by direct chemical or microbiological manipulation of compound (III), as it is shown in scheme 1.
For example, compounds of formula (I), wherein R² has the meaning of a 5-membered heterocycloalkyl having one O atom and optionally substituted at any carbon atom with one or two substituents which are the same or different, and independently selected from the group consisting of oxo and OH, and wherein R¹ has the meaning of methyl or -COOH, can be produced by stirring an organic hydroperoxide with compound (III) in the presence of iron trichloride in a solvent such as pyridine.
Alternatively, the methods disclosed in WO2016/091776 A1 can be applied to produce compounds of general formula (I).

Furthermore, compounds of formula (I) wherein R² has the meaning of C₃-C₄-alkyl optionally substituted with one or two substituents which are the same or different and independently selected from the group consisting of OH and -COOH, can be produced by incubation of compound (III) with Actinobacteria in microbiological growth medium at temperatures between 25 and 30 °C.
The scheme and procedures described below illustrate synthetic routes to the compounds of general formula (I) of the invention and are not intended to be limiting. Specific examples are described in the subsequent paragraphs.

Two routes for the preparation of compounds of general formula (I) are described in scheme 1. 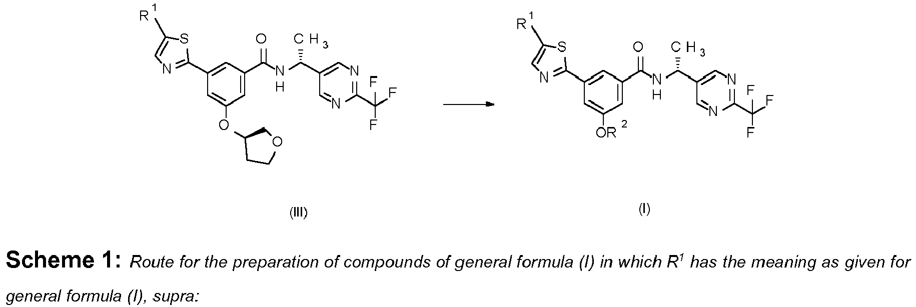
*a) preparation of compounds of formula (I) with R² having the meaning of C₃-C₄-alkyl optionally substituted with one or two substituents which are the same or different and independently selected from the group consisting of OH and -COOH through microbiological syntheses with Streptomyces in growth medium at temperatures between 25 and 30 °C.*
*b) preparation of compounds of formula (I) with R² having the meaning of 5-membered heterocycloalkyl having one O atom and optionally substituted at any carbon atom with one or two substituents which are the same or different, and independently selected from the group consisting of oxo and OH chemical synthesis with iron (III) trichloride in combination with tert-butyl hydroperoxide in pyridine.*

In accordance with a second aspect, the present invention covers methods of preparing compounds of general formula (I) as defined *supra,* culturing a microorganism in a culture medium, incubating this culture with a compound of general formula (III) and isolating the formed compound of general formula (I) from the medium.
In accordance with a further embodiment of the second aspect, the present invention covers methods of preparing compounds of general formula (I) as defined supra, said methods comprising the step of cultivation of Actinobacteria as microorganism in a culture medium, incubating this culture with a compound of general formula (III) and isolating the formed compound of general formula (I) from the medium.
In accordance with a further embodiment of the second aspect, the present invention covers methods of preparing compounds of general formula (I) as defined supra, said methods comprising the step of cultivation of *Streptomyces* bacteria as microorganism in a culture medium, incubating this culture with a compound of general formula (III) and isolating the formed compound of general formula (I) from the medium.
In accordance with a further embodiment of the second aspect, the present invention covers methods of preparing compounds of general formula (I) as defined supra, said methods comprising the step of cultivation of *Streptomyces roseochromogenus* as microorganism in a culture medium, incubating this culture with a compound of general formula (III) and isolating the formed compound of general formula (I) from the medium.
In accordance with a further embodiment of the second aspect, the present invention covers methods of preparing compounds of general formula (I) as defined supra, said methods comprising the step of cultivation of *Streptomyces albulus* as microorganism in a culture medium, incubating this culture with a compound of general formula (III) and isolating the formed compound of general formula (I) from the medium.

The compounds of general formula (I) of the present invention can be converted to any salt, preferably pharmaceutically acceptable salts, as described herein, by any method which is known to the person skilled in the art. Similarly, any salt of a compound of general formula (I) of the present invention can be converted into the free compound, by any method which is known to the person skilled in the art.
Compounds of general formula (I) of the present invention demonstrate a valuable pharmacological spectrum of action and pharmacokinetic profile if supported by data, both of which could not have been predicted. Compounds of the present invention have surprisingly been found to effectively inhibit the P2X3 receptor and it is possible therefore that said compounds be used for the treatment or prophylaxis of diseases, preferably neurogenic disorders in humans and animals.
Compounds of the present invention can be utilized to inhibit, block, reduce, decrease, etc., pharmacological mechanism. This method comprises administering to a mammal in need thereof, including a human, an amount of a compound of this invention, or a pharmaceutically acceptable salt, isomer, metabolite, hydrate, solvate or ester thereof; which is effective to treat the disorder.
In particular, the compounds of the present invention are suitable for the treatment and/ or prophylaxis of neurogenic disorders like genitourinary, gastrointestinal, respiratory, cardiovascular disease associated with autonomic imbalance caused by increased chemoreceptor sensitivity, and pain-related diseases, conditions and disorders.
The inventive compounds can therefore be used in medicaments for treatment and/or prophylaxis of the following diseases:
- gynecological diseases and related symptoms selected from the group consisting of dysmenorrhea (primary and secondary dysmenorrhea), dyspareunia, endometriosis, adenomyosis, endometriosis-associated pain, endometriosis-associated proliferation, pelvic hypersensitivity, and endometriosis-associated symptoms, wherein said symptoms are in particular dysuria or dyschezia;
- urinary tract disease states and related symptoms selected from the group consisting of bladder outlet obstruction, urinary incontinence conditions, reduced bladder capacity, increased frequency of micturition, urge incontinence, stress incontinence, bladder hyperreactivity, benign prostatic hypertrophy, prostatic hyperplasia, prostatitis, detrusor hyperreflexia, pelvic hypersensitivity, urethritis, prostatitis, prostatodynia, cystitis, Interstitial cystitis, idiopathic bladder hypersensitivity, overactive bladder, and symptoms related to overactive bladder wherein said symptoms are increased urinary frequency, nocturia, urinary urgency or urge incontinence;
- pain selected from the group consisting of acute, chronic, inflammatory and neuropathic pain;
- inflammatory pain selected from the group consisting of low back pain surgical pain, visceral pain, dental pain, periodontitis, premenstrual pain, endometriosis-associated pain, pain associated with fibrotic diseases, central pain, pain due to burning mouth syndrome, pain due to burns, pain due to migraine, cluster headaches, pain due to nerve injury, pain due to neuritis, neuralgias, pain due to poisoning, pain due to ischemic injury, pain due to interstitial cystitis, cancer pain, pain due to viral, parasitic or bacterial infections, pain due to traumatic nerve-injury, pain due to post-traumatic injuries (including fractures and sport injuries), pain due to trigeminal neuralgia, pain associated with small fiber neuropathy, pain associated with diabetic neuropathy, postherpetic neuralgia, chronic lower back pain, neck pain phantom limb pain, pelvic pain syndrome, chronic pelvic pain, neuroma pain, complex regional pain syndrome, pain associated with gastrointestinal distension, chronic arthritic pain and related neuralgias, and pain associated with cancer, Morphine-resistant pain, pain associated with chemotherapy, HIV and HIV treatment-induced neuropathy; and pain associated with diseases or disorders selected from the group consisting of hyperalgesia, allodynia, functional bowel disorders (such as irritable bowel syndrome), and arthritis (such as osteoarthritis, rheumatoid arthritis and ankylosing spondylitis);
- pain-associated diseases or disorders selected from the group consisting of hyperalgesia, allodynia, functional bowel disorders (including irritable bowel syndrome), gout, arthritis (including osteoarthritis, rheumatoid arthritis and ankylosing spondylitis), burning mouth syndrome, burns, migraine or cluster headaches, nerve injury, traumatic nerve injury, post-traumatic injuries (including fractures and sport injuries), neuritis, neuralgias, poisoning, ischemic injury, interstitial cystitis, cancer, trigeminal neuralgia, small fiber neuropathy, diabetic neuropathy, chronic arthritis and related neuralgias, HIV and HIV treatment-induced neuropathy, pruritus, impaired wound healing, and disease of the skeleton including degeneration of the joints;
- Epilepsy, partial and generalized seizures;
- Respiratory disorders selected from the group consisting of chronic obstructive pulmonary disorder (COPD), asthma, bronchospasm, pulmonary fibrosis, acute cough, and chronic cough including chronic idiopathic and chronic refractory cough;
- Gastrointestinal disorders selected from the group consisting of irritable bowel syndrome (IBS), inflammatory bowel disease (IBD), biliary colic and other biliary disorders, renal colic, diarrhea-dominant IBS, gastroesophageal reflux, gastrointestinal distension, and Crohn's disease;
- neurodegenerative disorders selected from the group consisting of Alzheimer's disease, Multiple Sclerosis, Parkinson's disease, Brain ischemia, and traumatic brain injury;
- breathing disorders, Cheyne Stokes respiration, central and obstructive sleep apnea, cardiovascular disease, hypertension, resistant hypertension, and heart failure, which are associated with autonomic imbalance caused by increased chemoreceptor sensitivity
- myocardial infarction, lipid disorders;
- pruritus.

The present invention also provides methods of treating neurogenic disorders, in particular for the treatment of the above-mentioned diseases and disorders.
These disorders have been well characterized in humans, but also exist with a similar etiology in other mammals, and can be treated by administering pharmaceutical compositions of the present invention.
The term "treating" or "treatment" as used in the present text is used conventionally, e.g., the management or care of a subject for the purpose of combating, alleviating, reducing, relieving, improving the condition of a disease or disorder, such as neurogenic disorders or diseases.
The compounds of the present invention can be used in therapy and prevention, i.e. prophylaxis, of neurogenic diseases, conditions and disorders.
The compounds of the present invention can be used in particular in the therapy and prevention, i.e. prophylaxis, of genitourinary, gastrointestinal, respiratory, cardiovascular disease associated with autonomic imbalance caused by increased chemoreceptor sensitivity, and pain-related diseases, conditions and disorders.
In accordance with a further aspect, the present invention covers compounds of general formula (I), as described *supra,* or stereoisomers, hydrates, solvates, salts thereof, particularly pharmaceutically acceptable salts thereof, or mixtures of same, for use in the treatment or prophylaxis of diseases, in particular neurogenic disorders.
The pharmaceutical activity of the compounds according to the invention can be explained by their activity as P2X3 inhibitors.
In accordance with a further aspect, the present invention covers the use of compounds of general formula (I), as described *supra,* or stereoisomers, hydrates, solvates, and salts thereof, particularly pharmaceutically acceptable salts thereof, or mixtures of same, for the treatment or prophylaxis of diseases, in particular neurogenic disorders, particularly of genitourinary, gastrointestinal, respiratory, cardiovascular disease associated with autonomic imbalance caused by increased chemoreceptor sensitivity, and pain-related diseases, conditions and disorders.
The term "genitourinary disease, condition and disorder" as used in the present text is used conventionally, e.g., for diseases, conditions and disorders of the genitourinary system. In particular, it is used in the present text for diseases of female pelvic organs, disorders of female genital tract and diseases of urinary system. In other words, term "genitourinary disease, condition and disorder" as used in the present text is used conventionally, e.g., for gynaecological and urinary tract disease, condition and disorder.
In accordance with a further aspect, the present invention covers the use of a compound of formula (I), described *supra,* or a stereoisomer, a hydrate, a solvate, or a salt thereof, particularly a pharmaceutically acceptable salt thereof, or a mixture of same, for the prophylaxis or treatment of diseases, in particular of gynecological diseases, particularly dysmenorrhea (primary and secondary dysmenorrhea), dyspareunia, endometriosis, adenomyosis, endometriosis-associated pain, endometriosis-associated proliferation, pelvic hypersensitivity, and endometriosis-associated symptoms, wherein said symptoms are in particular dysuria or dyschezia.
In accordance with a further aspect, the present invention covers the use of compounds of general formula (I), as described *supra,* or stereoisomers, hydrates, solvates, and salts thereof, particularly pharmaceutically acceptable salts thereof, or mixtures of same, in a method of treatment or prophylaxis of diseases, in particular urinary tract disease states, particularly bladder outlet obstruction, urinary incontinence conditions, reduced bladder capacity, increased frequency of micturition, urge incontinence, stress incontinence, bladder hyperreactivity, benign prostatic hypertrophy, prostatic hyperplasia, prostatitis, detrusor hyperreflexia, pelvic hypersensitivity, urethritis, prostatitis, prostatodynia, cystitis, Interstitial cystitis, idiopathic bladder hypersensitivity, overactive bladder, and symptoms related to overactive bladder wherein said symptoms are increased urinary frequency, nocturia, urinary urgency or urge incontinence.

In accordance with a further aspect, the present invention covers use of a compound of general formula (I), as described *supra,* or stereoisomers, hydrates, solvates, and salts thereof, particularly pharmaceutically acceptable salts thereof, or mixtures of same, for the preparation of a pharmaceutical composition, preferably a medicament, for the prophylaxis or treatment of pain-related disease, condition and disorder.
The term "pain-related disease, condition and disorder" or "pain-associated disease, condition and disorder" and similar as used in the present text is used conventionally, e.g., for acute, chronic, inflammatory and neuropathic pain diseases, conditions and disorders.

In accordance with a further aspect, the present invention covers use of a compound of general formula (I), as described *supra,* or stereoisomers, hydrates, solvates, and salts thereof, particularly pharmaceutically acceptable salts thereof, or mixtures of same, for the preparation of a pharmaceutical composition, preferably a medicament, for the prophylaxis or treatment of pain-related diseases, conditions and disorders, in particular acute, chronic, inflammatory and neuropathic pain disorders, particularly inflammatory pain selected from the group consisting of low back pain surgical pain, visceral pain, dental pain, periodontitis, premenstrual pain, endometriosis-associated pain, pain associated with fibrotic diseases, central pain, pain due to burning mouth syndrome, pain due to burns, pain due to migraine, cluster headaches, pain due to nerve injury, pain due to neuritis, neuralgias, pain due to poisoning, pain due to ischemic injury, pain due to interstitial cystitis, cancer pain, pain due to viral, parasitic or bacterial infections, pain due to traumatic nerve-injury, pain due to post-traumatic injuries (including fractures and sport injuries), pain due to trigeminal neuralgia, pain associated with small fiber neuropathy, pain associated with diabetic neuropathy, postherpetic neuralgia, chronic lower back pain, neck pain phantom limb pain, pelvic pain syndrome, chronic pelvic pain, neuroma pain, complex regional pain syndrome, pain associated with gastrointestinal distension, chronic arthritic pain and related neuralgias, and pain associated with cancer, Morphine-resistant pain, pain associated with chemotherapy, HIV and HIV treatment-induced neuropathy; and pain associated with diseases or disorders selected from the group consisting of hyperalgesia, allodynia, functional bowel disorders (such as irritable bowel syndrome), and arthritis (such as osteoarthritis, rheumatoid arthritis and ankylosing spondylitis).
In accordance with a further aspect, the present invention covers use of a compound of general formula (I), as described *supra,* or stereoisomers, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular of respiratory disorders, particularly chronic obstructive pulmonary disorder (COPD), asthma, bronchospasm, pulmonary fibrosis, acute cough, and chronic cough including chronic idiopathic and chronic refractory cough.
In accordance with a further aspect, the present invention covers use of a compound of general formula (I), as described *supra,* or stereoisomers, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular of gastrointestinal disorders, particularly irritable bowel syndrome (IBS), inflammatory bowel disease (IBD), biliary colic and other biliary disorders, renal colic, diarrhea-dominant IBS, gastroesophageal reflux, gastrointestinal distension, and Crohn's disease.

In accordance with a further aspect, the present invention covers use of a compound of general formula (I), as described *supra,* or stereoisomers, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular for the treatment of breathing disorders, Cheyne Stokes respiration, central and obstructive sleep apnea, cardiovascular disease, hypertension, resistant hypertension, and heart failure, which are related to increased activity of P2X3 receptors.

In accordance with a further aspect, the present invention covers use of a compound of general formula (I), as described *supra,* or stereoisomers, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular of pain-associated diseases or disorders, particularly hyperalgesia, allodynia, functional bowel disorders (including irritable bowel syndrome), gout, arthritis (including osteoarthritis, rheumatoid arthritis and ankylosing spondylitis), burning mouth syndrome, burns, migraine or cluster headaches, nerve injury, traumatic nerve injury, post-traumatic injuries (including fractures and sport injuries), neuritis, neuralgias, poisoning, ischemic injury, interstitial cystitis, cancer, trigeminal neuralgia, small fiber neuropathy, diabetic neuropathy, chronic arthritis and related neuralgias, HIV and HIV treatment-induced neuropathy, pruritus, impaired wound healing, and disease of the skeleton including degeneration of the joints.

In accordance with a further aspect, the present invention covers a method of treatment or prophylaxis of diseases, in particular neurogenic disorders, particularly genitourinary, gastrointestinal, respiratory, cardiovascular disease associated with autonomic imbalance caused by increased chemoreceptor sensitivity, and pain-related diseases, conditions and disorders, using an effective amount of a compound of general formula (I), as described *supra,* or stereoisomers, hydrates, solvates, and salts thereof, particularly pharmaceutically acceptable salts thereof, or mixtures of same.
In accordance with a further aspect, the present invention covers pharmaceutical compositions, in particular a medicament, comprising a compound of general formula (I), as described *supra,* or a stereoisomer, a tautomer, an N-oxide, a hydrate, a solvate, a salt thereof, particularly a pharmaceutically acceptable salt, or a mixture of same, and one or more excipients), in particular one or more pharmaceutically acceptable excipient(s). Conventional procedures for preparing such pharmaceutical compositions in appropriate dosage forms can be utilized.

The present invention furthermore covers pharmaceutical compositions, in particular medicaments, which comprise at least one compound according to the invention, conventionally together with one or more pharmaceutically suitable excipients, and to their use for the above mentioned purposes.
It is possible for the compounds according to the invention to have systemic and/or local activity. For this purpose, they can be administered in a suitable manner, such as, for example, via the oral, parenteral, pulmonary, nasal, sublingual, lingual, buccal, rectal, vaginal, dermal, transdermal, conjunctival, otic route or as an implant or stent.
For these administration routes, it is possible for the compounds according to the invention to be administered in suitable administration forms.

For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/ lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.
Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.
Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents.
The compounds according to the invention can be incorporated into the stated administration forms. This can be effected in a manner known per se by mixing with pharmaceutically suitable excipients. Pharmaceutically suitable excipients include, inter alia,
- fillers and carriers (for example cellulose, microcrystalline cellulose (such as, for example, Avicel®), lactose, mannitol, starch, calcium phosphate (such as, for example, Di-Cafos®)),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette®), sorbitan fatty acid esters (such as, for example, Span®), polyoxyethylene sorbitan fatty acid esters (such as, for example, Tween®), polyoxyethylene fatty acid glycerides (such as, for example, Cremophor®), polyoxethylene fatty acid esters, polyoxyethylene fatty alcohol ethers, glycerol fatty acid esters, poloxamers (such as, for example, Pluronic®),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol®); alginates, gelatine),
- disintegrants (for example modified starch, carboxymethylcellulose-sodium, sodium starch glycolate (such as, for example, Explotab®), cross- linked polyvinylpyrrolidone, croscarmellose-sodium (such as, for example, AcDiSol®)),
- flow regulators, lubricants, glidants and mould release agents (for example magnesium stearate, stearic acid, talc, highly-disperse silicas (such as, for example, Aerosil®)),
- coating materials (for example sugar, shellac) and film formers for films or diffusion membranes which dissolve rapidly or in a modified manner (for example polyvinylpyrrolidones (such as, for example, Kollidon®), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit®)),
- capsule materials (for example gelatine, hydroxypropylmethylcellulose),
- synthetic polymers (for example polylactides, polyglycolides, polyacrylates, polymethacrylates (such as, for example, Eudragit®), polyvinylpyrrolidones (such as, for example, Kollidon®), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.

The present invention furthermore relates to a pharmaceutical composition, which comprise at least one compound according to the invention, conventionally together with one or more pharmaceutically suitable excipient(s), and to their use according to the present invention.
In accordance with another aspect, the present invention covers pharmaceutical combinations, in particular medicaments, comprising at least one compound of general formula (I) of the present invention and at least one or more further active ingredients, in particular for the treatment and/or prophylaxis of neurogenic disorders, in particular of genitourinary, gastrointestinal, respiratory, cardiovascular disease associated with autonomic imbalance caused by increased chemoreceptor sensitivity, and pain-related diseases, conditions and disorders.
Particularly, the present invention covers a pharmaceutical combination, which comprises:
- one or more first active ingredients, in particular compounds of general formula (I) as defined *supra,* and
- one or more further active ingredients, suitable for the treatment of neurogenic disorders, genitourinary, gastrointestinal, respiratory, cardiovascular disease associated with autonomic imbalance caused by increased chemoreceptor sensitivity, and pain-related diseases, conditions and disorders.

The term "combination" in the present invention is used as known to persons skilled in the art, it being possible for said combination to be a fixed combination, a non-fixed combination or a kit-of-parts.
A "fixed combination" in the present invention is used as known to persons skilled in the art and is defined as a combination wherein, for example, a first active ingredient, such as one or more compounds of general formula (I) of the present invention, and a further active ingredient are present together in one unit dosage or in one single entity. One example of a "fixed combination" is a pharmaceutical composition wherein a first active ingredient and a further active ingredient are present in admixture for simultaneous administration, such as in a formulation. Another example of a "fixed combination" is a pharmaceutical combination wherein a first active ingredient and a further active ingredient are present in one unit without being in admixture.
A non-fixed combination or "kit-of-parts" in the present invention is used as known to persons skilled in the art and is defined as a combination wherein a first active ingredient and a further active ingredient are present in more than one unit. One example of a non-fixed combination or kit-of-parts is a combination wherein the first active ingredient and the further active ingredient are present separately. It is possible for the components of the non-fixed combination or kit-of-parts to be administered separately, sequentially, simultaneously, concurrently or chronologically staggered.
The compounds of the present invention can be administered as the sole pharmaceutical agent or in combination with one or more other pharmaceutically active ingredients where the combination causes no unacceptable adverse effects. The present invention also covers such pharmaceutical combinations. For example, the compounds of the present invention can be combined with known hormonal therapeutic agents.
The compounds of the present invention can be combined with therapeutic agents or active ingredients, that are already approved or that are still under development for the treatment and/ or prophylaxis of diseases, which are related to or mediated by P2X3 receptor. Such therapeutic agents or active ingredients are for example, but not limited, to 5-(2,4-Diamino-pyrimidin-5-yloxy)-4-isopropyl-2-methoxy-benzenesulfonamide (Gefapixant/ MK-7264/AF-219), (5-(5-iodo-2-isopropyl-4-methoxy-phenoxy)-pyrimidine-2,4-diamine (AF-353), 5-[2-isopropyl-4-methoxy-5-(methylsulfonyl)phenoxy]pyrimidine-2,4-diamine (AF-130), 2-[[4-amino-5-(5-iodo-4-methoxy-2-propan-2-ylphenoxy)-pyrimidin-2-yl]amino]propane-1,3-diol (AF-906), and (S)-methyl 2-((2-(2,6-difluoro-4-(methylcarbamoyl)-phenyl)-5-methyl-1H-benzo[d]imidazol-1-yl)methyl)morpholine-4-carboxylate (BLU-5937/ NEO 5937).
The compounds of the present invention can be combined with therapeutic agents or active ingredients, that are already approved or that are still under development for the treatment and/ or prophylaxis of diseases, which are related to other targets like NK1 inhibitors, for example 2-(R)-(4-Fluoro-2-methyl-phenyl)-4-(S)-((8aS)-6-oxohexahydropyrrolo[1,2-a]-pyrazin-2-yl)-piperidine-1-carboxylic acid [1-(R)-(3,5-bis-trifluoromethylphenyl)-ethyl]-methylamide (Orvepitant), 3-[(3aR,4R,5S,7aS)-5-{(1R)-1-[3,5-bis(trifluoromethyl)phenyl]ethoxy}-4-(4-fluorophenyl)octahydro-2H-isoindol-2-yl]cyclopent-2-en-1-one (Serlopitant), or nicotinic Acetylcholine modulators, for example N-(2-((3-pyridinyl)Methyl)-1-azabicyclo[2.2.2]oct-3-yl)benzofuran-2-carboxamide (Bradanicline/ ATA-101).

In particular, the compounds of the present invention can be administered in combination or as comedication with hormonal contraceptives. Hormonal contraceptives can be administered via oral, subcutaneous, transdermal, intrauterine or intravaginal route, for example as Combined Oral Contraceptives (COCs) or Progestin-Only-Pills (POPs) or hormone-containing devices like implants, patches or intravaginal rings.
COCs include but are not limited to birth control pills or a birth control method that includes a combination of an estrogen (estradiol) and a progestogen (progestin). The estrogenic part is in most of the COCs ethinyl estradiol. Some COCs contain estradiol or estradiol valerate.
Said COCs contain the progestins norethynodrel, norethindrone, norethindrone acetate, ethynodiol acetate, norgestrel, levonorgestrel, norgestimate, desogestrel, gestodene, drospirenone, dienogest, or nomegestrol acetate.
Birth control pills include for example but are not limited to Yasmin, Yaz, both containing ethinyl estradiol and drospirenone; Microgynon or Miranova containing levonorgestrel and ethinyl estradiol; Marvelon containing ethinyl estradiol and desogestrel; Valette containing ethinyl estradiol and dienogest; Belara and Enriqa containing ethinyl estradiol and chlormadinonacetate; Qlaira containing estradiol valerate and dienogest as active ingredients; and Zoely containing estradiol and normegestrol.

POPs are contraceptive pills that contain only synthetic progestogens (progestins) and do not contain estrogen. They are colloquially known as mini pills.
POPs include but are not limited to Cerazette containing desogestrel; Microlut containing levonorgestrel and Micronor containing norethindrone.
Other Progeston-Only forms are intrauterine devices (IUDs), for example Mirena containing levonorgestrel or injectables, for example Depo-Provera containing medroxyprogesterone acetate, or implants, for example Implanon containing etonogestrel.
Other hormone-containing devices with contraceptive effect which are suitable for a combination with the compounds of the present invention are vaginal rings like Nuvaring containing ethinyl estradiol and etonogestrel or transdermal systems like a contraceptive patch, for example Ortho-Evra or Apleek (Lisvy) containing ethinyl estradiol and gestodene.

A preferred embodiment of the present invention is the administration of a compound of general formula (I) in combination with a COC or a POP or other Progestin-Only forms as well as vaginal rings or contraceptive patches as mentioned above.

For the treatment and/ or prophylaxis of urinary tract diseases, the compounds of the present invention can be administered in combination or as comedication with any substance that can be applied as therapeutic agent in the following indications:
Urinary tract disease states associated with the bladder outlet obstruction; urinary incontinence conditions such as reduced bladder capacity, increased frequency of micturition, urge incontinence, stress incontinence, or bladder hyperreactivity; benign prostatic hypertrophy; prostatic hyperplasia; prostatitis; detrusor hyperreflexia; overactive bladder and symptoms related to overactive bladder wherein said symptoms are in particular increased urinary frequency, nocturia, urinary urgency or urge incontinence; pelvic hypersensitivity; urethritis; prostatitis; prostatodynia; cystitis, in particular interstitial cystitis; idiopathic bladder hypersensitivity.

For the treatment and/ or prophylaxis of overactive bladder and symptoms related to overactive bladder, the compounds of the present invention can be administered in combination or as comedication, independently or in addition to behavioral therapy like diet, lifestyle or bladder training, with anticholinergics like oxybutynin, tolterodine, propiverine, solifenacin, darifenacin, trospium, fesoterdine; β-3 agonists like mirabegron; neurotoxins like onabutolinumtoxin A; or antidepressants like imipramine, duloxetine.
For the treatment and/ or prophylaxis of interstitial cystitis, the compounds of the present invention can be administered in combination or as comedication, independently or in addition to behavioral therapy like diet, lifestyle or bladder training, with pentosans like elmiron; NSAIDS (Non-Steroidal Antiinflammatory Drugs), either unselective NSAIDS like ibuprofen, diclofenac, aspirin, naproxen, ketoprofen, indomethacin; as well as Cox-2 selective NSAIDS like Parecoxib, Etoricoxib, Celecoxib; antidepressants like amitriptyline, imipramine; or antihistamines like loratadine.

For the treatment and/ or prophylaxis of gynaecological diseases, the compounds of the present invention can be administered in combination or as comedication with any substance that can be applied as therapeutic agent in the following indications:
dysmenorrhea, including primary and secondary dysmenorrhea; dyspareunia; endometriosis; endometriosis-associated pain; endometriosis-associated symptoms, wherein said symptoms are in particular dysmenorrhea, dyspareunia, dysuria, or dyschezia.
For the treatment and/ or prophylaxis of dysmenorrhea, including primary and secondary dysmenorrhea; dyspareunia; endometriosis and endometriosis-associated pain, the compounds of the present invention can be administered in combination or as comedication with pain medicaments, in particular NSAIDS like ibuprofen, diclofenac, aspirin, naproxen, ketoprofen, indomethacin; as well as Cox-2 selective NSAIDS like Parecoxib, Etoricoxib, Celecoxib; or in combination with ovulation inhibiting treatment, in particular COCs as mentioned above or contraceptive patches like Ortho-Evra or Apleek (Lisvy); or with progestogenes like dienogest (Visanne); or with GnRH analogous, in particular GnRH agonists and antagonists, for example leuprorelin, nafarelin, goserelin, cetrorelix, abarelix, ganirelix, degarelix; or with androgens: danazol.
For the treatment and/ or prophylaxis of endometriosis and endometriosis-associated pain, the compounds of the present invention can be administered in combination or as comedication with GnRH antagonists like Elagolix, Linzagolix, or Relugolix.

For the treatment and/ or prophylaxis of endometriosis and endometriosis-associated pain, the compounds of the present invention can be administered in combination or as comedication with Selective Progesterone Receptor Modulators (SPRMs) or Progesterone antagonists like Vilaprisan, Ulipristal acetate, Telapristone, or Mifepristone.

For the treatment and/ or prophylaxis of diseases which are associated with pain, or pain syndromes, the compounds of the present invention can be administered in combination or as comedication with any substance that can be applied as therapeutic agent in the following indications:
pain-associated diseases or disorders like hyperalgesia, allodynia, functional bowel disorders (such as irritable bowel syndrome) and arthritis (such as osteoarthritis, rheumatoid arthritis and ankylosing spondylitis), burning mouth syndrome, burns, migraine or cluster headache, nerve injury, traumatic nerve injury, post-traumatic injuries (including fractures and sport injuries), neuritis, neuralgia, poisoning, ischemic injury, interstitial cystitis, trigeminal neuralgia, small fiber neuropathy, diabetic neuropathy, chronic arthritis and related neuralgias, HIV and HIV treatment-induced neuropathy.

The compounds of the present invention can be combined with other pharmacological agents and compounds that are intended to treat inflammatory diseases, inflammatory pain or general neuropathic pain conditions.
In addition to well-known medicaments which are already approved and on the market, the compounds of the present invention can be administered in combination with inhibitors of PTGES (prostaglandin E synthase), with inhibitors of IRAK4 (interleukin-1 receptor-associated kinase 4) and with antagonists of the prostanoid EP4 receptor (prostaglandin E2 receptor 4).
In particular, the compounds of the present invention can be administered in combination with pharmacological endometriosis agents, intended to treat inflammatory diseases, inflammatory pain or general neuropathic pain conditions and/or interfering with endometriotic proliferation and endometriosis associated symptoms, namely with inhibitors of Aldo-keto-reductase1C3 (AKR1C3) and with functional blocking antibodies of the prolactin receptor.

For the treatment and/ or prophylaxis of chronic cough and symptoms related to chronic cough, the compounds of the present invention can be administered in combination or as comedication with cough suppressants like dextromethorphan, benzonatate, codeine or hydrocodone; with inhalative agents to treat eosinophilic bronchitis, COPD or asthma like budesonide, beclomethasone, fluticasone, theophylline, ipatropiumbromid, montelukast or salbutamol; with drugs like proton pump inhibitors which are used to treat acid reflux, for example omeprazole, esomeprazole, lansoprazole, ranitidine, famotidine, cimetidine; and promotility agents such as metoclopramide; with nasal or topical glucocorticoids like fluticasone or mometasone or triamcinolone; or with oral antihistamines like loratadine, fexofenadine or cetirizine.

The compounds of the present invention can be combined with other pharmacological agents and compounds that are intended for the treatment, prevention or management of cancer.
In particular, the compounds of the present invention can be administered in combination with 131I-chTNT, abarelix, abiraterone, aclarubicin, ado-trastuzumab emtansine, afatinib, aflibercept, aldesleukin, alemtuzumab, Alendronic acid, alitretinoin, altretamine, amifostine, aminoglutethimide, Hexyl aminolevulinate,amrubicin, amsacrine, anastrozole, ancestim, anethole dithiolethione, angiotensin II, antithrombin III, aprepitant, arcitumomab, arglabin, arsenic trioxide, asparaginase, axitinib, azacitidine, basiliximab, belotecan, bendamustine, belinostat, bevacizumab, bexarotene, bicalutamide, bisantrene, bleomycin, bortezomib, buserelin, bosutinib, brentuximab vedotin, busulfan, cabazitaxel, cabozantinib, calcium folinate, calcium levofolinate, capecitabine, capromab, carboplatin, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, copanlisib, crisantaspase, cyclophosphamide, cyproterone, cytarabine, dacarbazine, dactinomycin, darbepoetin alfa, dabrafenib, dasatinib, daunorubicin, decitabine, degarelix, denileukin diftitox, denosumab, depreotide, deslorelin, dexrazoxane, dibrospidium chloride, dianhydrogalactitol, diclofenac, docetaxel, dolasetron, doxifluridine, doxorubicin, doxorubicin + estrone, dronabinol, eculizumab, edrecolomab, elliptinium acetate, eltrombopag, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, I-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, interferon alfa, interferon beta, interferon gamma, iobitridol, iobenguane (1231), iomeprol, ipilimumab, irinotecan, Itraconazole, ixabepilone, lanreotide, lapatinib, lasocholine, lenalidomide, lenograstim, lentinan, letrozole, leuprorelin, levamisole, levonorgestrel, levothyroxine sodium, lisuride, lobaplatin, lomustine, lonidamine, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, nabilone, nabiximols, nafarelin, naloxone + pentazocine, naltrexone, nartograstim, nedaplatin, nelarabine, neridronic acid, nivolumabpentetreotide, nilotinib, nilutamide, nimorazole, nimotuzumab, nimustine, nitracrine, nivolumab, obinutuzumab, octreotide, ofatumumab, omacetaxine mepesuccinate, omeprazole, ondansetron, oprelvekin, orgotein, orilotimod, oxaliplatin, oxycodone, oxymetholone, ozogamicine, p53 gene therapy, paclitaxel, palifermin, palladium-103 seed, palonosetron, pamidronic acid, panitumumab, pantoprazole, pazopanib, pegaspargase, PEG-epoetin beta (methoxy PEG-epoetin beta), pembrolizumab, pegfilgrastim, peginterferon alfa-2b, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib , regorafenib, risedronic acid, rhenium-186 etidronate, rituximab, romidepsin, romiplostim, romurtide, roniciclib , samarium (153Sm) lexidronam, sargramostim, satumomab, secretin, sipuleucel-T, sizofiran, sobuzoxane, sodium glycididazole, sorafenib, stanozolol, streptozocin, sunitinib, talaporfin, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib , valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

Furthermore, the compounds of the present invention can be combined with active ingredients, which are well known for the treatment of cancer-related pain and chronic pain. Such combinations include, but are not limited to NSAIDS (either unselective NSAIDS like ibuprofen, diclofenac, aspirin, naproxen, ketoprofen and indomethacin; and Cox-2 selective NSAIDS like Parecoxib, Etoricoxib and Celecoxib), step II opiods like codeine phosphate, dextropropoxyphene, dihydro¬codeine,Tramadol), step III opiods like morphine, fentanyl, buprenorphine, oxymorphone, oxycodone and hydromorphone; and other medications used for the treatment of cancer pain like steroids as Dexamethasone and methylprednisolone; bisphosphonates like Etidronate, Clodronate, Alendronate, Risedronate, and Zoledronate; tricyclic antidepressants like Amitriptyline, Clomipramine, Desipramine, Imipramine and Doxepin; class I antiarrhythmics like mexiletine and lidocaine; anticonvulsants like carbamazepine, Gabapentin, oxcarbazepine, phenytoin, pregabalin, topiramate, alprazolam, diazepam, flurazepam, pentobarbital and phenobarbital.

Based upon standard laboratory techniques known to evaluate compounds useful for the treatment of neurogenic, by standard toxicity tests and by standard pharmacological assays for the determination of treatment of the conditions identified above in mammals, and by comparison of these results with the results of known active ingredients or medicaments that are used to treat these conditions, the effective dosage of the compounds of the present invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.
The total amount of the active ingredient to be administered will generally range from about 0.001 mg/kg to about 200 mg/kg body weight per day, and preferably from about 0.01 mg/kg to about 50 mg/kg body weight per day. Clinically useful dosing schedules will range from one to three times a day dosing to once every four weeks dosing. In addition, it is possible for "drug holidays", in which a patient is not dosed with a drug for a certain period of time, to be beneficial to the overall balance between pharmacological effect and tolerability. It is possible for a unit dosage to contain from about 0.5 mg to about 400 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.
Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

Methods of testing for a particular pharmacological or pharmaceutical property are well known to persons skilled in the art.

The example testing experiments described herein serve to illustrate the present invention and the invention is not limited to the examples given.

### EXPERIMENTAL SECTION

The ¹H-NMR data of selected compounds are listed in the form of ¹H-NMR peaklists. Therein, for each signal peak the δ value in ppm is given, followed by the signal intensity, reported in round brackets. The δ value-signal intensity pairs from different peaks are separated by commas. Therefore, a peaklist is described by the general form: δ₁ (intensity₁), δ₂ (intensity₂), ... , δᵢ (intensityᵢ), ... , δₙ (intensityₙ).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A ¹H-NMR peaklist is similar to a classical ¹H-NMR readout, and thus usually contains all the peaks listed in a classical NMR interpretation. Moreover, similar to classical ¹H-NMR printouts, peaklists can show solvent signals, signals derived from stereoisomers of the particular target compound, peaks of impurities, ¹³C satellite peaks, and/or spinning sidebands. The peaks of stereoisomers, and/or peaks of impurities are typically displayed with a lower intensity compared to the peaks of the target compound (e.g., with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical ¹H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

Chemical names were generated using the ACD/Name software from ACD/Labs. In some cases generally accepted names of commercially available reagents were used in place of ACD/Name generated names.

The following table 1 lists the abbreviations used in this paragraph and in the Examples section as far as they are not explained within the text body. Other abbreviations have their meanings customary *per se* to the skilled person.

**Table 1: Abbreviations**

| The following table lists the abbreviations used herein. | |
|---|---|
| **Abbreviation** | **Meaning** |
| aq. | aqueous |
| br | broad (¹H-NMR signal) |
| conc. | concentrated |
| d | doublet |
| DAD | diode array detector |
| DCM | dichloromethane |
| dd | double-doublet |
| DIPEA | diisopropylethylamine |
| DMF | *N,N*-dimethylformamide |
| DMSO | dimethylsulfoxide |
| dt | double-triplet |
| EDTA | ethylenediaminetetraacetic acid |
| EtOH | ethanol |
| eq. | equivalent |
| h | hour(s) |
| HPLC | high performance liquid chromatography |
| LC-MS | liquid chromatography mass spectrometry |
| m | multiplet |
| min | minute(s) |
| MeCN | acetonitrile |
| MeOH | methanol |
| MS | mass spectrometry |
| NMR | nuclear magnetic resonance spectroscopy: chemical shifts (δ) are given in ppm. The chemical shifts were corrected by setting the DMSO signal to 2.50 ppm unless otherwise stated. |
| PDA | Photo Diode Array |
| Pluronic® PE 8100 | Pluronic PE types are low-foaming, nonionic surfactants. They are block copolymers in which the central polypropylene glycol group is flanked by two polyethylene glycol groups. PE 8100 conforms to the following structural formula: HO(CH₂CH₂O)ₓ(CH₂C(CH₃)HO)_{y}(CH₂CH₂O)_{z}H |
| | PE 8100 is a polypropylene glycol block copolymer with a molar mass of 2300 g/mol and 10 % polypropylene glycol in the molecule. |
| q | quartet |
| r.t. or rt or RT | room temperature |
| rac | racemic |
| Rt | retention time (as measured either with HPLC or UPLC) in minutes |
| s | singlet |
| sat. | saturated |
| SM | starting material |
| SQD | Single-Quadrupole-Detector |
| t | triplet |
| td | triple-doublet |
| TEA | triethylamine |
| TFA | trifluoroacetic acid |
| THF | tetrahydrofuran |
| UPLC | ultra performance liquid chromatography |

The various aspects of the invention described in this application are illustrated by the following examples, which are not meant to limit the invention in any way.

The example testing experiments described herein serve to illustrate the present invention and the invention is not limited to the examples given.

### EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartidges KP-Sil® or KP-NH® in combination with a Biotage autopurifier system (SP4® or Isolera Four®) and eluents such as gradients of hexane/ethyl acetate or DCM/methanol. In some cases, the compounds may be purified by preparative HPLC using for example a Waters autopurifier equipped with a diode array detector and/or online electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

### EXPERIMENTAL SECTION - EXAMPLES

### Example 1:

### {[(3R,5R)-5-Hydroxytetrahydrofuran-3-yl]oxy}-5-(5-methyl-1,3-thiazol-2-yl)-N-{(1R)-1-[2-(trifluoromethyl)pyrimidin-5-yl]ethyl}benzamide and {[(3R,5S)-5-hydroxytetrahydrofuran-3-yl]oxy}-5-(5-methyl-1,3-thiazol-2-yl)-N-{(1R)-1-[2-(trifluoromethyl)pyrimidin-5-yl]ethyl}benzamide

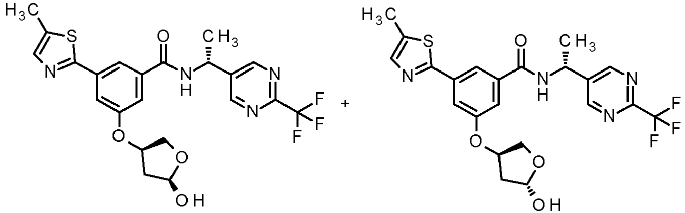

A mixture of 3-(5-methyl-1,3-thiazol-2-yl)-5-[(3R)-tetrahydrofuran-3-yloxy]-N-{(1R)-1-[2-(trifluoromethyl)pyrimidin-5-yl]ethyl}benzamide (200 mg, 0.42 mmol), and Iron(III) chloride (56 mg, 0.21 mmol) were added to a reaction vessel, pyridine was added (2 ml), and tert-butylhydroperoxide 70% mixture in water, 239 µl, 1.67 mmol) was added dropwise. The reaction vessel was sealed and the reaction left to stir for 48 hours at RT. Saturated Ethylenediaminetetraacetic acid solution (mono sodium salt, 15 ml) was added and the mixture stirred at RT for 10 minutes. Brine was added, and the aqueous phase was extracted with dichloromethane (100 ml), the organic phase was dried by passing it through a water repellent filter and the solvent removed under reduced pressure. The title compounds (2,4 mg, 1,2% yield) were obtained by purification using a Labomatic HD5000, Labocord-5000; Gilson GX-241, Labcol Vario 4000 system, with a Chiralpak ID 5µ 250x30mm column; A mobile phase of hexane:ethanol was used with a gradient of 25-50% (ethanol) over 15 min with a flow rate of 40.0 ml/min with detection using a UV wavelength of 325 nm.

### Analytical HPLC method:

Instrument: Waters Autopurification MS SingleQuad; Column: Waters XBrigde C18 5µ 100x30mm; eluent A: water + 0.1 vol % formic acid (99%), eluent B: acetonitrile; gradient: 0-5.5 min 5-100% B; flow 70 ml/min; temperature: 25 °C; DAD scan: 210-400 nm.LC-MS: Rₜ = 1.10 min; 495,27 (M+H)⁺
¹H NMR (600 MHz, DMSO-*d*₆) δ ppm 1.59 - 1.63 (m, 3 H) 2.13 - 2.21 (m, 1 H) 2.39 - 2.45 (m, 1 H) 2.52 - 2.55 (m, 3 H) 2.77 (t, J=6.10 Hz, 1 H) 3.32 (s, 1 H) 3.38 - 3.50 (m, 1 H) 3.79 (q, J=6.10 Hz, 1 H) 3.84 - 3.99 (m, 1 H) 3.88 (d, J=10.30 Hz, 1 H) 3.95 (dd, J=9.92, 3.43 Hz, 1 H) 4.10 - 4.15 (m, 1 H) 4.96 (t, J=5.34 Hz, 1 H) 5.06 - 5.35 (m, 1 H) 5.09 - 5.17 (m, 1 H) 5.24 - 5.33 (m, 1 H) 5.38 - 5.60 (m, 1 H) 5.41 - 5.44 (m, 1 H) 5.55 (q, J=4.58 Hz, 1 H) 6.20 - 6.34 (m, 1 H) 6.25 (d, J=4.58 Hz, 1 H) 6.30 (d, J=4.96 Hz, 1 H) 7.47 - 7.55 (m, 1 H) 7.59 - 7.71 (m, 1 H) 7.81 (m, 1 H) 7.91 - 7.94 (m, 1 H) 9.10 - 9.20 (m, 3 H)
1H-NMR (600 MHz, DMSO-d6) delta [ppm]: -0.006 (0.83), 0.005 (0.72), 0.785 (0.50), 0.797 (0.89), 0.810 (0.61), 0.825 (0.44), 0.836 (0.44), 0.842 (0.61), 0.854 (1.16), 0.865 (0.78), 1.008 (0.66), 1.032 (2.05), 1.043 (2.33), 1.086 (0.78), 1.146 (0.94), 1.157 (1.77), 1.170 (0.94), 1.182 (0.55), 1.207 (0.61), 1.234 (3.16), 1.259 (0.94), 1.262 (1.44), 1.285 (0.39), 1.296 (0.72), 1.590 (1.38), 1.602 (12.35), 1.614 (11.35), 1.835 (0.55), 1.858 (0.61), 2.163 (1.27), 2.168 (2.44), 2.174 (2.05), 2.177 (2.33), 2.183 (1.38), 2.386 (1.22), 2.389 (1.66), 2.391 (1.27), 2.421 (0.44), 2.520 (4.76), 2.523 (6.59), 2.525 (5.65), 2.544 (1.49), 2.614 (1.27), 2.617 (1.72), 2.619 (1.27), 2.759 (0.61), 2.769 (1.22), 2.779 (0.61), 3.321 (0.44), 3.377 (0.50), 3.784 (0.83), 3.793 (0.78), 3.873 (1.99), 3.890 (2.10), 3.942 (0.50), 3.948 (0.50), 3.959 (0.55), 3.964 (0.55), 4.112 (1.55), 4.119 (1.94), 4.128 (1.72), 4.136 (1.49), 4.142 (0.50), 4.956 (0.78), 5.101 (0.44), 5.110 (0.39), 5.248 (1.22), 5.255 (1.16), 5.261 (0.61), 5.273 (0.55), 5.285 (1.88), 5.297 (2.66), 5.308 (1.66), 5.320 (0.44), 5.426 (0.55), 5.539 (0.83), 5.547 (1.72), 5.553 (1.77), 5.561 (0.83), 6.242 (0.94), 6.250 (0.94), 6.291 (3.27), 6.299 (3.21), 7.334 (0.44), 7.433 (0.44), 7.474 (1.83), 7.478 (3.04), 7.480 (2.66), 7.492 (3.88), 7.495 (4.15), 7.499 (1.88), 7.503 (1.11), 7.505 (1.16), 7.509 (0.61), 7.622 (0.72), 7.624 (0.66), 7.642 (0.55), 7.650 (4.82), 7.652 (4.93), 7.677 (0.72), 7.679 (0.72), 7.707 (0.44), 7.710 (0.66), 7.713 (0.50), 7.793 (0.66), 7.819 (0.44), 7.822 (0.66), 7.927 (4.21), 7.929 (5.31), 7.931 (2.77), 8.243 (0.50), 8.245 (0.83), 8.248 (0.50), 8.321 (0.78), 9.110 (2.38), 9.121 (16.00), 9.130 (2.93), 9.170 (2.10), 9.182 (2.66), 9.194 (0.83), 10.078 (0.94).
¹³C NMR (151 MHz, DMSO-d6) δ ppm 0.18 11.77 20.83 20.88 37.81 38.95 40.08 40.19 40.26 40.59 45.35 56.86 69.83 70.30 76.43 77.55 78.81 79.03 79.24 97.48 97.58 114.66 115.75 117.53 118.82 120.64 134.78 134.84 135.07 135.10 136.21 136.23 140.40 141.88 153.65 153.89 156.81 156.84 157.51 164.27 164.30 165.11 165.15

### Example 2:

### 3-{[(2R)-1,4-dihydroxybutan-2-yl]oxy}-5-(5-methyl-1,3-thiazol-2-yl)-N-{(1R)-1-[2-(trifluoromethyl)pyrimidin-5-yl]ethyl}benzamide

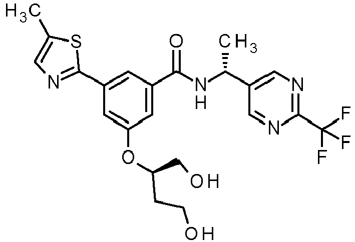

### Preculture generation

A DMSO cryo culture (0.2 mL) of *Streptomyces roseochromogenus* (CBS 41563) was added to a 100-mL Erlenmeyer flask containing sterile growth medium (20 mL) consisting of D-(+)-glucose monohydrate (10 g L⁻¹), yeast extract (1 g L⁻¹), beef extract (1 g L⁻¹) and tryptose (2 g L⁻¹) which had been adjusted to pH 7.2 with sodium hydroxide solution (16% in water) and had been sterilized at 121 °C for 20 minutes. After inoculation, the mixture was shaken on a rotation shaker (165 rpm) at 27 °C for 48 h. This preculture (10 mL per flask) was added to two 500-mL Erlenmeyer flasks containing the same sterile growth medium (100 mL per flask, prepared under the same conditions) and the flasks were shaken on a rotation shaker (rpm 165) at 27 °C for 72 h.

### Biotransformation

The preculture (50 mL per flask) of the 500-mL Erlenmeyer flasks was added to a 1L Biostat Q fermenter containing sterile growth medium (1 L) consisting of D-(+)-glucose monohydrate (10 g L⁻¹), yeast extract (1 g L⁻¹), beef extract (1 g L⁻¹) and tryptose (2 g L^{- 1}) which had been adjusted to pH 7.2 with sodium hydroxide solution. Silicon oil (0.05 mL) and Pluronic® PE 8100 (0.05 mL) sterilized at 121 °C for 30 minutes were added. Compound (III), i.e. 3-(5-methyl-1,3-thiazol-2-yl)-5-[(3R)-tetrahydrofuran-3-yloxy]-N-{(1R)-1-[2-(trifluoromethyl)pyrimidin-5-yl]ethyl}benzamide, (25.0 mg, 0.052 mmol) dissolved in DMF (2 mL) was added and the culture was stirred at 220 rpm at 27 °C with an aeration rate of 2.0 L/min. The culture was stirred at an oxygen partial pressure of 15% regulated by the stirring rate of up to 800 rpm. After 72 h the culture was harvested.

The preculture (50 mL each) of the 500-mL Erlenmeyer flasks was added to two 1L Biostat Q fermenters each containing sterile growth medium (1 L per fermenter) consisting of D-(+)-glucose monohydrate (10 g L⁻¹), yeast extract (1 g L⁻¹), beef extract (1 g L⁻¹) and tryptose (2 g L⁻¹) which had been adjusted to pH 7.2 with sodium hydroxide solution. Silicon oil (0.05 mL) and Pluronic® PE 8100 (0.05 mL) sterilized at 121 °C for 30 minutes were added. After 5 h compound (III), i.e. 3-(5-methyl-1,3-thiazol-2-yl)-5-[(3R)-tetrahydrofuran-3-yloxy]-N-{(1R)-1-[2-(trifluoromethyl)pyrimidin-5-yl]ethyl}benzamide, (25.0 mg, 0.052 mmol) dissolved in DMF (2 mL) was added and the culture was stirred at 220 rpm at 27 °C with an aeration rate of 2.0 L/min. The culture was stirred at an oxygen partial pressure of 15% regulated by the stirring rate of up to 800 rpm. After 59 or 67 h the culture was harvested.

The three culture broths of the biotransformations were combined and extracted with 4-methyl-2-pentanone. The organic layer was concentrated to give an oil (0.71 g) which was stirred at 50 °C in methanol (10 mL). The resulting solid was filtered off and the filtrate was concentrated to give an oil (0.30 g).

Another preculture was generated as described before.

The preculture (50 mL or 100 mL) of the 500-mL Erlenmeyer flasks was added to two 1L Biostat Q fermenters each containing sterile growth medium (1 L per fermenter) consisting of D-(+)-glucose monohydrate (10 g L⁻¹), yeast extract (1 g L⁻¹), beef extract (1 g L⁻¹) and tryptose (2 g L⁻¹) which had been adjusted to pH 7.2 with sodium hydroxide solution. Silicon oil (0.05 mL) and Pluronic® PE 8100 (0.05 mL) sterilized at 121 °C for 30 minutes were added. After 5 h compound (III), i.e. 3-(5-methyl-1,3-thiazol-2-yl)-5-[(3R)-tetrahydrofuran-3-yloxy]-N-{(1R)-1-[2-(trifluoromethyl)pyrimidin-5-yl]ethyl}benzamide (25.0 mg, 0.052 mmol) dissolved in DMF (2 mL) was added and the culture was stirred at 220 rpm at 27 °C with an aeration rate of 2.0 L/min. The culture was stirred at an oxygen partial pressure of 15% regulated by the stirring rate of up to 800 rpm. After 47 h the culture was harvested.

The preculture (100 mL) of the 500-mL Erlenmeyer flasks was added to a 1L Biostat Q fermenter containing sterile growth medium (1 L) consisting of D-(+)-glucose monohydrate (10 g L⁻¹), yeast extract (1 g L⁻¹), beef extract (1 g L⁻¹) and tryptose (2 g L^{- 1}) which had been adjusted to pH 7.2 with sodium hydroxide solution. Silicon oil (0.05 mL) and Pluronic® PE 8100 (0.05 mL) sterilized at 121 °C for 30 minutes were added. Compound (III), i.e. 3-(5-methyl-1,3-thiazol-2-yl)-5-[(3R)-tetrahydrofuran-3-yloxy]-N-{(1R)-1-[2-(trifluoromethyl)pyrimidin-5-yl]ethyl}benzamide (25.0 mg, 0.052 mmol) dissolved in DMF (2 mL) was added and the culture was stirred at 220 rpm at 27 °C with an aeration rate of 2.0 L/min. The culture was stirred at an oxygen partial pressure of 15% regulated by the stirring rate of up to 800 rpm. After 12 h an aqueous glucose solution (20%, 1 g/h) was added. After further 10 h an aqueous glucose solution (20%, 2 g/h) was added. After 52 h the culture was harvested.

The preculture (100 mL) of the 500-mL Erlenmeyer flasks was added to a 1L Biostat Q fermenter containing sterile growth medium (1 L) consisting of D-(+)-glucose monohydrate (10 g L⁻¹), yeast extract (1 g L⁻¹), beef extract (1 g L⁻¹) and tryptose (2 g L^{- 1}) which had been adjusted to pH 7.2 with sodium hydroxide solution. Silicon oil (0.05 mL) and Pluronic® PE 8100 (0.05 mL) sterilized at 121 °C for 30 minutes were added. After 5 h compound (III), i.e. 3-(5-methyl-1,3-thiazol-2-yl)-5-[(3R)-tetrahydrofuran-3-yloxy]-N-{(1R)-1-[2-(trifluoromethyl)pyrimidin-5-yl]ethyl}benzamide, (25.0 mg, 0.052 mmol) dissolved in DMF (2 mL) was added and the culture was stirred at 220 rpm at 27 °C with an aeration rate of 2.0 L/min. The culture was stirred at an oxygen partial pressure of 15% regulated by the stirring rate of up to 800 rpm. After 16 h an aqueous glucose solution (20%, 2 g/h) was added. After 47 h the culture was harvested.

The four culture broths of the biotransformations were combined and extracted with 4-methyl-2-pentanone. The organic layer was concentrated to give an oil (0.91 g) which was stirred at 50 °C in methanol (20 mL). The resulting solid was filtered off and the filtrate was concentrated to give an oil (0.75 g).

The two crude products were combined and further purified by flash chromatography using silica gel (dichloromethane/methanol gradient) and by preparative HPLC to give the title compound (21.0 mg, 90 % purity, 10 % yield).

### Preparative chiral HPLC method:

Instrument: Waters Autopurificationsystem; Column: Waters XBrigde C18 5µ 100x30mm; Eluent A: water + 0.2 vol-% aqueous ammonia (32%), Eluent B: acetonitrile; gradient: 0.00-0.50 min 30% B (25-70mL/min), 0.51-5.50 min 30-40% B (70mL/min), DAD scan: 210-400 nm.

### Analytical chiral HPLC method:

Instrument: Waters Acquity UPLCMS SingleQuad; Column: Acquity UPLC BEH C18 1.7 µm, 50x2.1mm; eluent A: water + 0.1 vol-% formic acid (99%), eluent B: acetonitrile; gradient: 0-1.6 min 1-99% B, 1.6-2.0 min 99% B; flow 0.8 mL/min; temperature: 60 °C; DAD scan: 210-400 nm.
LC-MS: Rₜ = 1.00 min; MS (ESIpos): m/z = 497 [M+H]⁺
¹H-NMR (400MHz, DMSO-d₆): δ [ppm]= 1.61 (d, 3H), 1.70 - 1.91 (m, 2H), 3.43 - 3.55 (m, 2H), 3.56 - 3.61 (m, 2H), 4.37 - 5.15 (m, 2H), 4.55 - 4.65 (m, 1H), 5.30 (br d, 1H), 7.55 (dd, 1H), 7.62 - 7.66 (m, 2H), 7.90 (t, 1H), 9.09 - 9.14 (m, 2H), 9.14 - 9.22 (m, 1H).

Screening various wild-type strains the following strains showed a formation of the title compound:
DSMZ: Deutsche Sammlung von Mikroorganismen und Zellkulturen
ATCC: American Type Culture Collection
NRRL: ARS Culture Collection
IFO=NBRC: Biological Resource Center, National Institute of technology and Evaluation
CBS: Centraalbureau voor Schimmelculture, Netherlands

| **Strain** | **Origin** |
|---|---|
| Streptomyces griseus | ATCC 10137 |
| Streptomyces albus | ATCC 3004 |
| Streptomyces griseocarneus | ATCC 12628 |
| Streptomyces viridis | ATCC 15732 |
| Streptomyces fulvissimus | NRRL B-1453 |
| Streptomyces halstedii | ATCC 13449 |
| Streptomyces vinaceus | ATCC 11861 |
| Streptomyces hydrogenans | ATCC 19631 |
| Streptomyces fradiae | IFO 3360 |
| Streptomyces roseochromogenus | IFO 3363 |
| Streptomyces roseochromogenus | ATCC 13400 |
| Streptomyces roseochromogenus | CBS 41563 |
| Streptomyces roseochromogenus | NRRL B-1233 |
| Streptomyces phaeochromogenes | ATCC 3338 |
| Streptomyces griseus | ATCC 13273 |
| Streptomyces toyocaensis | DSMZ 40030 |
| Streptomyces roseus | DSMZ 40076 |
| Streptomyces sulphureus | DSMZ 40104 |
| Streptomyces capreolus | DSMZ 40225 |
| Streptomyces catenulae | DSMZ 40258 |
| Streptomyces cavourensis | DSMZ 40300 |
| Streptomyces polychromogenus | DSMZ 40316 |
| Streptomyces flocculus | DSMZ 40327 |
| Streptomyces varsoviensis | DSMZ 40346 |
| Streptomyces albulus | DSMZ 40492 |
| Streptomyces alboflavus | DSMZ 40761 |
| Streptomyces griseus subsp. griseus | DSMZ 40695 |
| Streptomyces griseus subsp. griseus | ATCC 12648 |
| Streptomyces griseus subsp. griseus | ATCC 27001 |
| Streptomyces griseus subsp. griseus | ATCC 31591 |
| Pseudonocardia autotrophica | DSMZ 43085 |
| Lechevalieria aerocolonigenes | ATCC 39243 |
| Streptomyces roseochromogenus | NBRC 3411 |
| Streptomyces tubercidicus | DSMZ 41958 |
| Streptomyces tubercidicus | DSMZ 41959 |
| Streptomyces roseochromogenus | ATCC 13400 |
| Streptomyces platensis | ATCC 13865 |
| Streptomyces griseus | IFO 3102 |

### Example 3

### (3R)-4-hydroxy-3-[3-(5-methyl-1,3-thiazol-2-yl)-5-({(1R)-1-[2-(trifluoromethyl)-pyrimidin-5-yl]ethyl}carbamoyl)phenoxy]butanoic acid

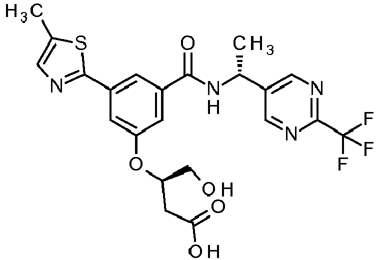

### Preculture generation

A DMSO cryo culture (0.2 mL) of *Streptomyces albulus* (DSMZ 40492) was added to a 100-mL Erlenmeyer flask containing sterile growth medium (20 mL) consisting of D-(+)-glucose monohydrate (10 g/L), yeast extract (1 g/L), beef extract (1 g/L) and tryptose (2 g/L) which had been adjusted to pH 7.2 with sodium hydroxide solution (16 % in water) and had been sterilized at 121 °C for 20 minutes. After inoculation, the mixture was shaken on a rotation shaker (165 rpm) at 27 °C for 48 h. This preculture (8 mL per flask) was added to two 2000-mL Erlenmeyer flasks containing the same sterile growth medium (1000 mL per flask, prepared under the same conditions) and the flasks were shaken on a rotation shaker (rpm 165) at 27 °C for 48 h.

### Biotransformation

The preculture (1000 mL per flask) of the 2000-mL Erlenmeyer flasks was added to a 10 L fermenter containing sterile growth medium (8.3 L) consisting of D-(+)-glucose monohydrate (4 g/L), yeast extract (4 g/L), malt extract (10 g/L) in demineralized water which had been adjusted to pH 7.2 with sodium hydroxide solution (16 % in water).

Silicon oil (0.5 mL) and Pluronic® PE 8100 (0.5 mL) sterilized at 121 °C for 40 minutes were added. Compound (III), i.e. 3-(5-methyl-1,3-thiazol-2-yl)-5-[(3R)-tetrahydrofuran-3-yloxy]-N-{(1R)-1-[2-(trifluoromethyl)pyrimidin-5-yl]ethyl}benzamide, (250 mg, 522 µmol) dissolved in DMF (10 mL) was added after 8 hours and the culture was stirred at 300 rpm at 27 °C with an aeration rate of 3.0 L/min. The culture was stirred at an oxygen partial pressure of 15 % regulated by the stirring rate of up to 800 rpm. After 91 hours the culture was harvested.

The culture broth was extracted with 4-methyl-2-pentanone for 17 hours and concentrated to give an oil (4.20 g) which was stirred at 50 °C in methanol. The resulting solid was filtered off and the filtrate was concentrated to give an oil (3.64 g). The culture broth was extracted again with 4-methyl-2-pentanone for 20 hours and concentrated to give an oil (1.47 g) which was stirred at 50 °C in methanol. The resulting solid was filtered off and the filtrate was concentrated to give an oil (0.85 g). The combined crude products were purified by flash chromatography using silica gel (dichloromethane/ methanol + 0.1 % ammonia gradient) and by preparative HPLC to give two batches of the title compound (20.0 mg and 17.6 mg).

### Analytical chiral HPLC method:

Instrument: Waters Acquity UPLCMS SingleQuad; Column: Acquity UPLC BEH C18 1.7 µm, 50x2.1 mm; Eluent A: Water + 0.2 Vol-% aqueous ammonia (32 %), Eluent B: Acetonitrile; Gradient: 0-1.6 min 1-99 % B, 1.6-2.0 min 99 % B; Flow 0.8 mL/min; Temperature: 60 °C; DAD scan: 210-400 nm.

### Preparative HPLC method for batch 1:

Instrument: Waters Autopurificationsystem; Column: Waters XBrigde C18 5µ 100x30 mm; Eluent A: Water + 0.2 Vol-% aqueous ammonia (32 %), Eluent B: Acetonitrile; Gradient: 0.00-0.50 min 17 % B (25-70 mL/min), 0.51-10.00 min 17 - 37 % B (70 mL/min), DAD scan: 210-400 nm.

### Preparative HPLC method for batch 2:

Instrument: Waters Autopurificationsystem; Column: Waters XBrigde C18 5µ 100x30 mm; Eluent A: Water + 0.2 Vol-% aqueous ammonia (32 %), Eluent B: Acetonitrile; Gradient: 0.00-0.50 min 7 % B (25-70 mL/min), 0.51-10.00 min 7 - 27 % B (70 mL/min), DAD scan: 210-400 nm.

### Additional preparative HPLC method for both batches:

Instrument: Waters Autopurificationsystem; Column: XBrigde C18 5µ, 100x30 mm; eluent A: water + 0.1 vol % formic acid; eluent B: acetonitrile; gradient: 0.0-0.5 min 25 % B (35-70 mL/min), 0.5-5.5 min 25-70 % B; flow: 70 mL/min; temperature: 25 °C; DAD scan: 210-400 nm.
LC-MS: Rₜ = 0.98 min; MS (ESIpos): m/z = 511 [M+H]⁺
¹H-NMR (400MHz, DMSO-d₆): δ [ppm]= 1.60 (d, 3H), 2.53 - 2.62 (m, 1H), 2.67 - 2.75 (m, 1H), 3.54 - 3.64 (m, 2H), 4.77 - 4.84 (m, 1H), 5.29 (quin, 1H), 7.55 (dd, 1H), 7.60 - 7.63 (m, 1H), 7.64 (d, 1H), 7.91 (t, 1H), 9.11 (s, 2H), 9.18 (d, 1H), 12.3 (s, 1H).
¹H-NMR (400 MHz, DMSO-d6) δ [ppm]: 1.594 (6.62), 1.612 (6.62), 2.518 (4.49), 2.522 (2.74), 2.539 (16.00), 2.563 (0.93), 2.583 (1.50), 2.604 (1.45), 2.678 (0.52), 2.684 (1.33), 2.695 (1.52), 2.724 (0.93), 2.736 (0.82), 3.547 (0.41), 3.559 (0.48), 3.576 (1.87), 3.588 (3.23), 3.600 (1.97), 3.616 (0.44), 3.629 (0.41), 4.791 (0.78), 4.803 (0.96), 4.811 (0.89), 4.823 (0.76), 5.276 (0.95), 5.294 (1.45), 5.312 (0.95), 7.542 (1.84), 7.546 (2.37), 7.548 (2.56), 7.552 (2.19), 7.612 (2.26), 7.616 (2.76), 7.622 (1.97), 7.643 (4.86), 7.646 (4.88), 7.914 (2.52), 7.918 (4.39), 7.921 (2.35), 9.114 (12.72), 9.173 (1.80), 9.191 (1.76).

### Example 4

### 2-{3-[(3R)-tetrahydrofuran-3-yloxy]-5-({(1R)-1-[2-(trifluoromethyl)pyrimidin-5-yl]ethyl}carbamoyl)phenyl}-1,3-thiazole-5-carboxylic acid

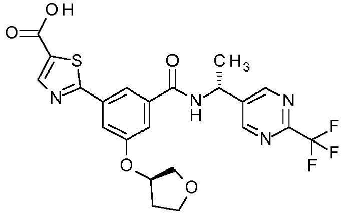

A mixture of 3-(5-methyl-1,3-thiazol-2-yl)-5-[(3R)-tetrahydrofuran-3-yloxy]-N-{(1R)-1-[2-(trifluoromethyl)pyrimidin-5-yl]ethyl}benzamide (200 mg, 0.42 mmol), and Iron(III) chloride (56 mg, 0.21 mmol) were added to a reaction vessel, pyridine was added (2 ml), and tert-butylhydroperoxide 70% mixture in water, 239 µl, 1.67 mmol) was added dropwise. The reaction vessel was sealed and the reaction left to stir for 48 hours at RT. Saturated Ethylenediaminetetraacetic acid solution (mono sodium salt, 15 ml) was added and the mixture stirred at RT for 10 minutes. Brine was added, and the aqueous phase was extracted with dichloromethane (100 ml), the organic phase was dried by passing it through a water repellent filter and the solvent removed under reduced pressure. The title compound (10.5 mg, 4.9 % yield) was obtained by purification using preparative HPLC using a Waters Autopurification system, equipped with a Waters XBrigde C18 5µ 100x30mm column; Eluent A: Water + 0.1 Vol-% formic acid (99%), Eluent B: Acetonitril; with the following gradient; 0.00-0.50 min 25% B (25->70mL/min), 0.51-7.50 min 25-45% B (70mL/min),

### Analytical HPLC method:

Instrument: Waters Acquity UPLCMS Single Quad; column: BEH C 18 1.7 50x2.1mm; Eluent A: water + 0.05 % formic acid (99%); Eluent B: acetonitrile + 0.05 % formic acid (99%); gradient: 0-0.2 2% B, 0.2-1.7 2-90% B, 1.7-1.9 90% B; 1.9-2.0 90-2%B, 2.0-2.5 2%B, flow 1.3 ml/min; temperature: 60°C; DAD scan: 200-400 nm.
LC-MS: Rₜ = 0.96 min; MS (ESIpos): m/z = 509 [M+H]⁺
1H NMR (500 MHz, DMSO-d6) δ ppm 1.61 (d, 3 H) 1.98 - 2.04 (m, 1 H) 2.22 - 2.31 (m, 1 H) 3.76 - 3.94 (m, 4 H) 5.23 - 5.33 (m, 2 H) 7.58 (s, 1 H) 7.65 (s, 1 H) 8.07 (s, 1 H) 8.40 (s, 1 H) 9.12 (s, 2 H) 9.21 (d, 1 H) 13.35 -14.10 (br. s, 1 H)
¹H-NMR (500 MHz, DMSO-d6) δ [ppm]: -0.006 (0.41), 0.000 (11.38), 0.007 (0.39), 1.235 (0.21), 1.482 (0.36), 1.609 (8.81), 1.623 (8.88), 1.987 (0.51), 1.998 (0.76), 2.012 (0.92), 2.024 (0.91), 2.036 (0.60), 2.235 (0.43), 2.248 (0.60), 2.251 (0.96), 2.263 (1.31), 2.279 (1.23), 2.291 (0.82), 2.307 (0.41), 2.361 (0.75), 2.365 (1.03), 2.369 (0.75), 2.522 (3.43), 2.526 (2.56), 2.544 (2.43), 2.635 (0.78), 2.639 (1.05), 2.643 (0.75), 3.768 (0.76), 3.777 (0.85), 3.784 (2.06), 3.793 (2.04), 3.801 (1.21), 3.810 (1.07), 3.843 (2.72), 3.859 (3.14), 3.873 (2.17), 3.889 (0.80), 3.910 (2.22), 3.919 (2.57), 3.930 (1.60), 3.939 (1.40), 5.240 (1.10), 5.249 (1.79), 5.260 (1.14), 5.276 (0.37), 5.290 (1.35), 5.304 (2.06), 5.318 (1.33), 5.332 (0.32), 7.579 (2.59), 7.584 (3.45), 7.586 (2.84), 7.655 (2.84), 7.659 (3.55), 7.663 (2.49), 8.078 (4.88), 8.142 (0.20), 8.319 (0.46), 8.407 (3.57), 9.129 (16.00), 9.206 (2.43), 9.220 (2.33).

### EXPERIMENTAL SECTION - BIOLOGICAL ASSAYS

Examples were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values or median values calculated utilizing data sets obtained from testing of one or more synthetic batch.

The *in vitro* activity of the compounds of the present invention can be demonstrated in the following assay:

### Intracellular calcium measurement to assess antagonist activity at human P2X3 receptors

A fluorescent imaging plate reader (FLEX/FLIPR station; Molecular Devices) was used to monitor intracellular calcium levels using the calcium-chelating dye Fluo-4 (Molecular Probes). The excitation and emission wavelengths used to monitor fluorescence were 470-495 nm and 515-575 nm, respectively. Cells expressing purinergic receptors P2X3 (human) were plated at a density of 15,000 cells/well in collagen-coated 384-well plates approximately 20 hours before beginning of the assay. On the day of the assay, 20 µL of loading buffer (Hank's balanced salt solution, 20 mM HEPES, 0.5 mM CaCI2, 0.5 mM MgCI2, 0.1% BSA, 5 mM probenecid, 10 mM D-glucose monohydrate, 2 µM Fluo-4, and 5 units/mL hexokinase, pH=7.4) was added and cells dye-loaded for 90 min at 37°C. The dye supernatant was removed and replaced with 45 µL probenecid buffer (Hank's balanced salt solution, 20 mM HEPES, 0.5 mM CaCI2, 0.5 mM MgCI2, 0.1% BSA, 5 mM probenecid, 10 mM D-glucose monohydrate, pH=7.4). The test compound was added in a volume of 5 µL and allowed to incubate for 30 min at 37°C. The final assay DMSO concentration is 1%. The agonist, α,β-Me-ATP, was added in a volume of 20 µL at a concentration representing the EC80 value. The fluorescence was measured for an interval of 90 sec at 2 sec intervals and analysed based on the increase in peak relative fluorescence units (RFU) compared to the basal fluorescence. Peak fluorescence was used to determine the response to agonist obtained at each concentration of test compound by the following equation: $% Reponse = 100 * \left({RFU}_{\left(test compound\right)}-{RFU}_{\left(control\right)}\right) / \left({RFU}_{\left(DMSO\right)}-{RFU}_{\left(control\right)}\right)$

The examples were tested in triplicates per plate and mean values were plotted in Excel XLFit to determine IC50 values at the human P2X3 receptors, percentage of maximal inhibition and the Hill coefficient.

**Table 2: IC₅₀ values of examples in Intracellular calcium measurement to assess antagonist activity at human P2X3 receptors**

| **Example** | **Target IC₅₀ [nM]** |
|---|---|
| example 11 of WO2016/091776 A1 | 6.0 |
| 1 | 20 |
| 2 | 6,8 |
| 3 | 13 |

### Solubilty

Examples were tested for its solubility in aqueous media (pH 6.5). Examples were synthesized one or more times in accordance with the above-described protocols . When synthesized more than once, data from solubility assays represent average values or median values calculated utilizing data sets obtained from testing of one or more synthetic batch.

The high throughput screening method to determine aqueous drug solubility is based on Thomas Onofrey and Greg Kazan, Performance and correlation of a 96-well high throughput screening method to determine aqueous drug solubility, Millipore Corporation Application Note, 2003; Lit. No. AN1731EN00.

The assay was run in a 96-well plate format. Each well was filled with an individual compound.
All pipetting steps were performed using a robot platform.
100 µl of a 10 mmolar solution of drug in DMSO were concentrated by vacuum centrifugation and resolved in 10 µl DMSO. 990 µl phosphate buffer pH 6.5 were added. The content of DMSO amounts to 1%. The multititer plate was put on a shaker and mixed for 24 hrs at room temperature. 150 µl of the suspension were transferred to a filtration plate. After filtration using a vacuum manifold, the filtrate was diluted with a 1:1 mixture of water and DMSO to 1:400 and 1:8000. A second microtiter plate with 20 µl of a 10 mM solution of drug in DMSO served for calibration. Two concentrations (1.25 nM and 2.5 nM) were prepared by dilution in DMSO / water 1:1 and used for calibration. Filtrate and calibration plates were quantified by HPLC-MS/MS.

Chromatographic conditions were as follows:

| | |
|---|---|
| HPLC column: | Ascentis Express C18 4.6 x 30 mm 2.7 µm |
| Injection volume: | 1 µl |
| Flow: | 1.5 ml/min |
| Mobile phase: | A: Water / 0.05% HCOOH |
| | B: Acetonitrile / 0.05% HCOOH |
| | 0 min → 95%A 5%B |
| | 0.75 min → 5%A 95%B |
| | 2.75 min → 5%A 95%B |
| | 2.76 min → 95%A 5%B |
| | 3 min → 95%A 5%B |

The areas of sample- and calibration injections were determined by using mass spectrometry software (AB SCIEX: Discovery Quant 2.1.3. and Analyst 1.6.1). The solubility value was obtained by interpolation from the calibration curve.
The significantly improved solubility of examples 1, 2 and 3 compared to example 11 of WO2016/091776 A1 is shown in table 3.

**Table 3: Aqueous solubility of example compounds**

| **Example** | **Aqueous Solubility pH 6.5 [mg/L]** |
|---|---|
| example 11 of WO2016/091776 A1 | 2.9 |
| 1 | 46.0 |
| 2 | 96.5 |
| 3 | 1190 |

### Metabolic stability

Examples were tested for its metabolic stability in human liver microsomes. Examples were measured one or more times. When measured more than once, data from metabolic stability assays represent average values or median values calculated utilizing data sets obtained from testing of one or more synthetic batch.

*In vitro* metabolic stability was determined by incubating the a solution of test compounds in dimethylsulfoxide (DMSO) and acetonitrile at 1 µM in a suspension of liver microsomes in 100 mM phosphate buffer, pH7.4 (NaH₂PO₄ x H₂O + Na₂HPO₄ x 2H₂O) and at a protein concentration of 0.5 mg/mL at 37° C. The microsomes were activated by adding a co-factor mix containing 8 mM Glucose-6-Phosphat, 4 mM MgCl2; 0.5 mM NADP and 1 IU/ml G-6-P-Dehydrogenase in phosphate buffer, pH 7.4. The metabolic assay was started shortly afterwards by adding the test compound to the incubation at a final volume of 1 mL. Organic solvent was limited to ≤0.01 % dimethylsulfoxide (DMSO) and ≤1% acetonitril. During incubation, the microsomal suspensions were continuously shaken at 580 rpm and aliquots were taken at 2, 8, 16, 30, 45 and 60 min, to which equal volumes of cold methanol were immediately added. Samples were frozen at -20°C over night, subsequently centrifuged for 15 minutes at 3000 rpm and the supernatant was analyzed with an Agilent 1200 HPLC-system with LCMS/MS detection.

The half-life of a test compound was determined from the concentration-time plot. From the half-life the intrinsic clearances were calculated. Together with the additional parameters liver blood flow, specific liver weight and microsomal protein content the hepatic *in vivo* blood clearance (CL) and the maximal oral bioavailability (Fₘₐₓ) were calculated for the different species. The hepatic *in vivo* blood clearance (CL_{blood}) and the maximal oral bioavailability (Fₘₐₓ) was calculated using the following formulae: CL'_{intrinsic} [ml/(min*kg)] = kₑₗ [1/min] / ((mg protein / volume of incubation [ml]) ^{∗} fu,_{inc}) ^{∗} (mg protein / liver weight [g]) ^{∗} (specific liver weight [g liver /kg body weight]); CL_{blood} well-stirred [L/(h^{∗}kg)] = (QH [L/(h^{∗}kg)] ^{∗} fu,_{blood} ^{∗} CL'_{intrinsic} [L/(h^{∗}kg)]) / (QH [L/(h^{∗}kg)] + fu,_{blood} ^{∗} CL'_{intrinsic} [L/(h^{∗}kg)]); Fmax = 1-CL_{blood} / QH and using the following parameter values: QH (Liver blood flow): 1.32 L/h/kg (human), 2.1 L/h/kg (dog), 4.2 L/h/kg (rat); specific liver weight: 21 g/kg (human), 39 g/kg (dog), 32 g/kg (rat); microsomal protein content: 40 mg/g. fu,_{inc} and fu,_{blood} is taken as 1 with fu,_{inc} being the unbound fraction (ie the non-protein bound fraction) in the incubation experiment, and fu,_{blood} being the unbound fraction in blood.

The significantly improved metabolic stability of example 2 and 3 of the present invention compared to example 11 of WO2016/091776 A1 is shown in table 4.

**Table 4: Metabolic stability of example compounds**

| **Example** | **CL** _{blood} **[L/h/kg]** | **F**ₘₐₓ**[%]** |
|---|---|---|
| example 11 of WO2016/091776 A1 | 0.52/0.27 | 61 /79 |
| 1 | 0.87 | 34 |
| 2 | 0.001 | 100 |
| 3 | 0.001 | 100 |

## Claims

**1.** A compound of general formula (I): 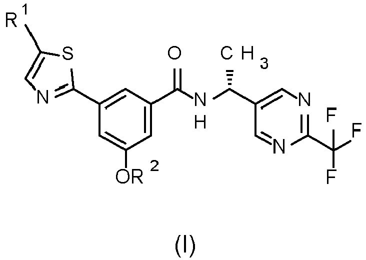 in which
R¹ represents methyl or -COOH,
R² represents C₃-C₄-alkyl optionally substituted with one or two substituents which are the same or different and independently selected from the group consisting of OH and -COOH, or 5-membered heterocycloalkyl having one O atom and optionally substituted at any carbon atom with one or two substituents which are the same or different, and independently selected from the group consisting of oxo and OH,
or stereoisomers, hydrates, solvates, salts thereof, or mixtures of same, as described and defined herein.

**2.** The compound according to claim 1, wherein R¹ represents methyl.

**3.** The compound according to claim 1 or 2, wherein
R² represents C₄-alkyl optionally substituted with one or two groups of OH, or 5-membered heterocycloalkyl having one or two O atom and optionally substituted at any carbon atom with one or two substituents which are the same or different, and selected from the group oxo and OH.

**4.** The compound according to claim 1, 2 or 3, wherein
R² represents C₃-alkyl optionally substituted with OH and COOH.

**5.** The compound according to any one of claims 1 to 4, wherein
R² represents C₄-alkyl optionally substituted with two OH.

**6.** The compound according to any one of claims 1 to 5, wherein R² represents C(CH₂OH)(CH₂)₂OH.

**7.** The compound according to any one of claims 1 to 6, wherein
R² represents tetrahydrofuranyl optionally substituted at any carbon atom with OH.

**8.** The compound according to to any one of claims 1 to 7, wherein
R¹ represents methyl, and
R² represents C(CH₂OH)(CH₂)₂OH.

**7.** The compound according to to any one of claims 1 to 8, wherein which is selected from the group consisting of:
3-{[(2R)-1,4-dihydroxybutan-2-yl]oxy}-5-(5-methyl-1,3-thiazol-2-yl)-N-{(1R)-1-[2-(trifluoromethyl)pyrimidin-5-yl]ethyl}benzamide,
rel-3-{[(3R,5R)-5-Hydroxytetrahydrofuran-3-yl]oxy}-5-(5-methyl-1,3-thiazol-2-yl)-N-{(1R)-1-[2-(trifluoromethyl)pyrimidin-5-yl]ethyl}benzamide,
{[(3R,5R)-5-Hydroxytetrahydrofuran-3-yl]oxy}-5-(5-methyl-1,3-thiazol-2-yl)-N-{(1R)-1-[2-(trifluoromethyl)pyrimidin-5-yl]ethyl}benzamide,
{[(3R,5S)-5-hydroxytetrahydrofuran-3-yl]oxy}-5-(5-methyl-1,3-thiazol-2-yl)-N-{(1R)-1-[2-(trifluoromethyl)pyrimidin-5-yl]ethyl}benzamide,
(3R)-4-hydroxy-3-[3-(5-methyl-1,3-thiazol-2-yl)-5-({(1R)-1-[2-(trifluoromethyl)pyrimidin-5-yl]ethyl}carbamoyl)phenoxy]butanoic acid,
2-{3-[(3R)-tetrahydrofuran-3-yloxy]-5-({(1R)-1-[2-(trifluoromethyl)pyrimidin-5-yl]ethyl}carbamoyl)phenyl}-1,3-thiazole-5-carboxylic acid.
or a stereoisomer, a hydrate, a solvate, or a salt thereof, or a mixture of same.

**8.** A compound of general formula (I) according to any one of claims 1 to 7, or an enantiomer, diastereomer, racemate, hydrate, solvate, or a pharmaceutically acceptable salt thereof, or a mixture thereof, for use in the treatment of a disease.

**9.** The compound for use according to claim 8, wherein the disease is a neurogenic disorder, such as genitourinary, gastrointestinal, respiratory, cardiovascular disease associated with autonomic imbalance caused by increased chemoreceptor sensitivity, and pain-related diseases.

**10.** Use of a compound of general formula (I) according to any one of claims 1 to 7, or an enantiomer, diastereomer, racemate, hydrate, solvate, or a pharmaceutically acceptable salt thereof, or a mixture thereof, for the preparation of a medicament for the prophylaxis or treatment of a disease.

**11.** The compound for use according to claim 9, wherein the genitourinary disease is selected from the group consisting of dysmenorrhea, dyspareunia, endometriosis, adenomyosis, endometriosis-associated pain, endometriosis-associated proliferation, pelvic hypersensitivity, dysuria, dyschezia.

**12.** The compound for use according to claim 9, wherein the genitourinary disease is selected from the group consisting of bladder outlet obstruction, urinary incontinence conditions, reduced bladder capacity, increased frequency of micturition, urge incontinence, stress incontinence, bladder hyperreactivity, benign prostatic hypertrophy, prostatic hyperplasia, prostatitis, detrusor hyperreflexia, pelvic hypersensitivity, urethritis, prostatitis, prostatodynia, cystitis, Interstitial cystitis, idiopathic bladder hypersensitivity, overactive bladder, and symptoms related to overactive bladder wherein said symptoms are increased urinary frequency, nocturia, urinary urgency or urge incontinence

**13.** The compound for use according to claim 9, wherein the respiratory disease is selected from the group consisting of chronic obstructive pulmonary disorder (COPD), asthma, bronchospasm, pulmonary fibrosis, acute cough, and chronic cough including chronic idiopathic and chronic refractory cough;

**14.** A pharmaceutical composition comprising a compound of general formula (I) according to any one of claims 1 to 7, or an enantiomer, diastereomer, racemate, hydrate, solvate, a pharmaceutically acceptable salt thereof, or a mixture thereof, and one or more pharmaceutically acceptable excipients.

**15.** A pharmaceutical combination according to claim 14, wherein further active ingredients are selected from the group consisting of cough suppressants, NSAIDS (Non-Steroidal Antiinflammatory Drug), Combined Oral Contraceptives (COC), GnRAH antagonists, Selective Progesterone Receptor Modulators (SPRMs), Progesterone antagonists, P2X3 inhibitors, NK1 inhibitors and nicotinic Acetylcholine modulators.
